# EUROPEAN PATENT SPECIFICATION

(11) **EP 4 169 090 B1**
(45) Date of publication and mention of the grant of the patent: **10.09.2025**
(21) Application number: 21733809.4
(22) Date of filing: 18.06.2021
(51) Int. Cl.: H10K 85/60, C07C 255/35, C07C 255/37, C07C 255/51, C07D 213/61, C07D 213/84, C07D 239/26, C07D 251/24, H10K 101/30, H10K 50/19

(54) **ORGANIC ELECTRONIC DEVICE COMPRISING A COMPOUND OF FORMULA (I), DISPLAY DEVICE COMPRISING THE ORGANIC ELECTRONIC DEVICE AS WELL AS COMPOUNDS OF FORMULA (I) FOR USE IN ORGANIC ELECTRONIC DEVICES**
ORGANISCHE ELEKTRONISCHE VORRICHTUNG MIT EINER VERBINDUNG DER FORMEL (I), ANZEIGEVORRICHTUNG MIT DER ORGANISCHEN ELEKTRONISCHEN VORRICHTUNG SOWIE VERBINDUNGEN DER FORMEL (I) ZUR VERWENDUNG IN ORGANISCHEN ELEKTRONISCHEN VORRICHTUNGEN
DISPOSITIF ÉLECTRONIQUE ORGANIQUE COMPRENANT UN COMPOSÉ DE FORMULE (I), DISPOSITIF D'AFFICHAGE COMPRENANT LE DISPOSITIF ÉLECTRONIQUE ORGANIQUE AINSI QUE COMPOSÉS DE FORMULE (I) DESTINÉS À ÊTRE UTILISÉS DANS DES DISPOSITIFS ÉLECTRONIQUES ORGANIQUES

(30) Priority: 22.06.2020 EP 20181386; 22.06.2020 EP 20181398; 22.06.2020 EP 20181408; 22.10.2020 EP 20203457; 22.10.2020 EP 20203463; 22.10.2020 EP 20203460; 22.10.2020 EP 20203458; 22.10.2020 EP 20203447; 14.06.2021 WO PCT/EP2021/065949
(43) Date of publication of application: 26.04.2023
(73) Proprietor: Novaled GmbH, 01099 Dresden (DE)
(72) Inventor: NÜLLEN, Max Peter, 01099 Dresden (DE); SCHULZE, Benjamin, 01099 Dresden (DE); WUDARCZYK, Jakob Jacek, 01099 Dresden (DE)
(74) Representative: Michalski Hüttermann & Partner Patentanwälte mbB
(86) International application number: PCT/EP2021/066610
(87) International publication number: WO 2021/259791

(56) References cited:
- EP-A1- 3 034 489
- EP-A1- 3 382 770
- WO-A1-2019/168368

## Description

### Technical Field

The present invention relates to an organic electronic device comprising a compound of formula (I) and a display device comprising the organic electronic device. The invention further relates to novel compounds of formula (I) which can be of use in organic electronic devices.

### Background Art

Organic electronic devices, such as organic light-emitting diodes OLEDs, which are self-emitting devices, have a wide viewing angle, excellent contrast, quick response, high brightness, excellent operating voltage characteristics, and color reproduction. A typical OLED comprises an anode, a hole transport layer HTL, an emission layer EML, an electron transport layer ETL, and a cathode, which are sequentially stacked on a substrate. In this regard, the HTL, the EML, and the ETL are thin films formed from organic compounds.

When a voltage is applied to the anode and the cathode, holes injected from the anode move to the EML, via the HTL, and electrons injected from the cathode move to the EML, via the ETL. The holes and electrons recombine in the EML to generate excitons. When the excitons drop from an excited state to a ground state, light is emitted. The injection and flow of holes and electrons should be balanced, so that an OLED having the above-described structure has excellent efficiency and/or a long lifetime.

Performance of an organic light emitting diode may be affected by characteristics of the semiconductor layer, and among them, may be affected by characteristics of compounds of formula (I) which are contained in the semiconductor layer. Compounds for organic optoelectronic devices and devices comprising these compounds are inter alia disclosed in the WO 2019 / 168368 A1, EP 3 382 770 A1 and EP3 034 489 A1.

There remains a need to improve performance of organic semiconductor materials, semiconductor layers, as well as organic electronic devices thereof, in particular to achieve improved operating voltage stability over time through improving the characteristics of the compounds comprised therein.

### DISCLOSURE

An aspect of the present invention provides an organic electronic device comprising an anode layer, a cathode layer and a charge generation layer, wherein the charge generation layer comprises a p-type charge generation layer and a n-type charge generation layer, wherein the p-type charge generation layer comprises a compound of formula (I) 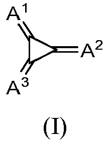 whereby A¹ is selected from formula (II) 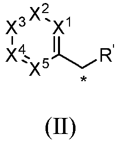
X¹ is selected from CR¹ or N;
X² is selected from CR² or N;
X³ is selected from CR³ or N;
X⁴ is selected from CR⁴ or N;
X⁵ is selected from CR⁵ or N;
whereby when any of R¹, R², R³, R⁴ and R⁵ is present, then the corresponding X¹, X², X³, X⁴ and X⁵ is not N;
wherein "*" denotes the binding position.
with the proviso that
   - R¹ (if present) is selected from D or H;
   - at least one of R², R³, R⁴ is present and for each of the R², R³, R⁴ that are present, the corresponding σ^{x} is > 0. 33, with σ^{X} being the Hammett constant of R^{x};
A² and A³ are independently selected from formula (III) 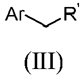
wherein Ar is independently selected from substituted or unsubstituted C₆ to C₁₈ aryl and substituted or unsubstituted C₂ to C₁₈ heteroaryl, wherein the substituents on Ar are independently selected from CN, partially or perfluorinated C₁ to C₆ alkyl, halogen, Cl, F, D;
and R' is selected from Ar, substituted or unsubstituted C₆ to C₁₈ aryl or C₃ to C₁₈ heteroaryl, partially flurorinated or perfluorinated C₁ to C₈ alkyl, halogen, F or CN; whereby R⁵ (if present) is selected from D or H.

It should be noted that throughout the application and the claims any Aⁿ, Bⁿ, Rⁿ, Xⁿ etc. always refer to the same moieties, unless otherwise noted.

In the present specification, when a definition is not otherwise provided, "substituted" refers to one substituted with a deuterium, C₁ to C₁₂ alkyl and C₁ to C₁₂ alkoxy.

However, in the present specification "aryl substituted" refers to a substitution with one or more aryl groups, which themselves may be substituted with one or more aryl and/or heteroaryl groups.

Correspondingly, in the present specification "heteroaryl substituted" refers to a substitution with one or more heteroaryl groups, which themselves may be substituted with one or more aryl and/or heteroaryl groups.

In the present specification, when a definition is not otherwise provided, an "alkyl group" refers to a saturated aliphatic hydrocarbyl group. The alkyl group may be a C₁ to C₁₂ alkyl group. More specifically, the alkyl group may be a C₁ to C₁₀ alkyl group or a C₁ to C₆ alkyl group. For example, a C₁ to C₄ alkyl group includes 1 to 4 carbons in alkyl chain, and may be selected from methyl, ethyl, propyl, iso-propyl, n-butyl, iso-butyl, sec-butyl, and tert-butyl.

Specific examples of the alkyl group may be a methyl group, an ethyl group, a propyl group, an isopropyl group, a butyl group, an iso-butyl group, a sec-butyl group, a tert-butyl group, a pentyl group, a hexyl group.

The term "cycloalkyl" refers to saturated hydrocarbyl groups derived from a cycloalkane by formal abstraction of one hydrogen atom from a ring atom comprised in the corresponding cycloalkane. Examples of the cycloalkyl group may be a cyclopropyl group, a cyclobutyl group, a cyclopentyl group, a cyclohexyl group, a methylcyclohexyl group, an adamantly group and the like.

The term "hetero" is understood the way that at least one carbon atom, in a structure which may be formed by covalently bound carbon atoms, is replaced by another polyvalent atom. Preferably, the heteroatoms are selected from B, Si, N, P, O, S; more preferably from N, P, O, S.

In the present specification, "aryl group" refers to a hydrocarbyl group which can be created by formal abstraction of one hydrogen atom from an aromatic ring in the corresponding aromatic hydrocarbon. Aromatic hydrocarbon refers to a hydrocarbon which contains at least one aromatic ring or aromatic ring system. Aromatic ring or aromatic ring system refers to a planar ring or ring system of covalently bound carbon atoms, wherein the planar ring or ring system comprises a conjugated system of delocalized electrons fulfilling Hückel's rule. Examples of aryl groups include monocyclic groups like phenyl or tolyl, polycyclic groups which comprise more aromatic rings linked by single bonds, like biphenyl, and polycyclic groups comprising fused rings, like naphtyl or fluoren-2-yl.

Analogously, under heteroaryl, it is especially where suitable understood a group derived by formal abstraction of one ring hydrogen from a heterocyclic aromatic ring in a compound comprising at least one such ring.

Under heterocycloalkyl, it is especially where suitable understood a group derived by formal abstraction of one ring hydrogen from a saturated cycloalkyl ring in a compound comprising at least one such ring.

The term "fused aryl rings" or "condensed aryl rings" is understood the way that two aryl rings are considered fused or condensed when they share at least two common sp²-hybridized carbon atoms.

The term "cyano moiety" refers to a CN substituent.

In the present specification, the single bond refers to a direct bond.

The term "n-type charge generation layer" is sometimes in the art also named n-CGL or electron generation layer and is intended to include the both.

The term "p-type charge generation layer" is sometimes in the art also named p-CGL or hole generation layer and is intended to include the both.

The term "free of", "does not contain", "does not comprise" does not exclude impurities which may be present in the compounds prior to deposition. Impurities have no technical effect with respect to the object achieved by the present invention.

The term "contacting sandwiched" refers to an arrangement of three layers whereby the layer in the middle is in direct contact with the two adjacent layers.

The terms "light-absorbing layer" and "light absorption layer" are used synonymously.

The terms "light-emitting layer", "light emission layer" and "emission layer" are used synonymously.

The terms "OLED", "organic light-emitting diode" and "organic light-emitting device" are used synonymously.

The terms "anode", "anode layer" and "anode electrode" are used synonymously.

The terms "cathode", "cathode layer" and "cathode electrode" are used synonymously.

The term "top emission device" is understood to mean an organic electronic device wherein the light is emitted through the cathode layer.

The term "bottom emission device" is understood to mean an organic electronic device wherein the light is emitted through the substrate.

The term "σ" and/or "Hammett constant" especially means and/or refers to the values given in Table I of Hansch et al. Chem. Rev. 1991, 65, 165-195. It should be noted that in the context of this application, the σ-values are differentiated only whether they are in meta-or para-position to the bonding to the cyclopropane core, i.e. the σ-values - if applicable - for R² and R⁴ are identical (and usually different from those at R³). Also it is not differentiated whether one or more nitrogen atoms are in the ring (i.e. whether any of X², X³, X⁴ and X⁵ is N); the σ-values - if applicable - for R², R³ and R⁴ do not change.

In the following Table 1 σ-values (as taken from Hansch et al. Chem. Rev. 1991, 65, 165-195) for commonly present moieties are given:

**Table 1: σₘ and σₚ values for various substituents**

| σ-values | H | F | CF₃ | CN | NO₂ | CF₂CF₃ | (CF₂)₃CF₃ |
|---|---|---|---|---|---|---|---|
| σ-values for para-substitution | 0 | 0.06 | 0.54 | 0.66 | 0.78 | 0.52 | 0.52 |
| σ-values for meta substitution | 0 | 0.34 | 0.43 | 0.56 | 0.71 | 0.47 | 0.47 |

In the specification, hole characteristics refer to an ability to donate an electron to form a hole when an electric field is applied and that a hole formed in the anode may be easily injected into the emission layer and transported in the emission layer due to conductive characteristics according to a highest occupied molecular orbital (HOMO) level.

In addition, electron characteristics refer to an ability to accept an electron when an electric field is applied and that electrons formed in the cathode may be easily injected into the emission layer and transported in the emission layer due to conductive characteristics according to a lowest unoccupied molecular orbital (LUMO) level.

### Advantageous Effects

Surprisingly, it was found that the organic electronic device according to the invention solves the problem underlying the present invention by enabling devices in various aspects superior over the organic electroluminescent devices known in the art, in particular with respect to operating voltage over lifetime.

According to one embodiment of the present invention, the p-type charge generation layer comprises a compound of formula (IV) 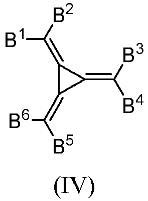 whereby B¹ is selected from formula (V) 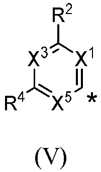 B³ and B⁵ are Ar and B², B⁴ and B⁶ are R³.

According to one embodiment, the p-type charge generation layer comprises a composition comprising a compound of formula (IV) and at least one compound of formula (IVa) to (IVd)

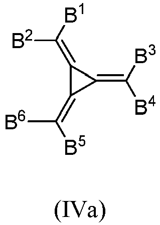

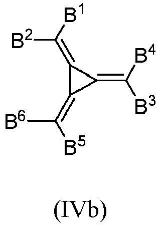

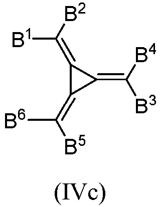

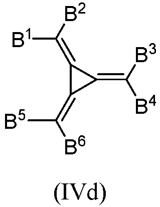

In case the p-type charge generation layer comprises such a composition, throughout this application text the term "compound of formula (I)" shall also intend to include the composition as described above.
According to one embodiment of the present invention, for at least one of the R², R³, R⁴ that are present, the corresponding σ^{x} is > 0. 35, with σ^{x} being the Hammett constant of R^{x};
According to one embodiment of the present invention, for each of the R², R³, R⁴ that are present, the corresponding σ^{x} is > 0. 35, with σ^{x} being the Hammett constant of R^{x};
According to one embodiment of the present invention, the compound of formula (I) comprises less than nine cyano moieties.
According to one embodiment of the present invention, the compound of formula (I) comprises between 3 and 8 cyano moieties.
According to one embodiment of the present invention, the compound of formula (I) comprises between 6 and 8 cyano moieties.
According to one embodiment of the present invention, whereby the LUMO of the compound of formula (I) is ≤ -5.05 eV, when calculated with the program package TURBOMOLE V6.5 (TURBOMOLE GmbH, Litzenhardtstrasse 19, 76135 Karlsruhe, Germany) by applying the hybrid functional B3LYP with a 6-31G* basis set in the gas phase, preferably in the range of ≤ -5.1 and ≥ -5.7 eV.
According to one embodiment of the present invention, σ^{total} > 0.5, with σ^{total} = σ² + σ³ + σ⁴ and σ^{x} being the Hammett constant of Rⁿ (if present).
According to one embodiment of the present invention, σ^{total} is > 0.6.
According to one embodiment of the present invention, σ^{total} is > 0.8.
According to one embodiment of the present invention, A² and A³ are identical.
According to one embodiment of the present invention, at least one from A² and A³ is identical to A¹.
According to one embodiment of the present invention, A¹, A² and A³ are identical.
According to one embodiment of the present invention, A¹ is different from A² and/or A³.
According to one embodiment of the present invention, R², R³, R⁴ (if present) are independently selected from NO₂, CN, halogen, Cl, F, partially fluorinated or perfluorinated alkyl, partially fluorinated or perfluorinated C₁ to C₁₀ alkyl, partially fluorinated or perfluorinated alkoxy, partially fluorinated or perfluorinated C₁ to C₆ alkoxy.
According to one embodiment of the present invention, R², R³, R⁴ (if present) are independently selected from NO₂, CN, F and CF₃.
According to one embodiment of the present invention, R' is CN.
According to one embodiment of the present invention, formula (II) is chosen out of one of the following moieties :

| Moiety | σ² | σ³ | σ⁴ | σ^{total} |
|---|---|---|---|---|
| 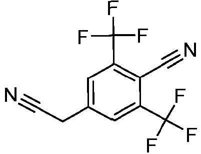 | 0.43 | 0.66 | 0.43 | 1.52 |
| 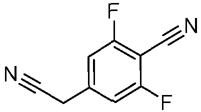 | 0.34 | 0.66 | 0.34 | 1.34 |
| 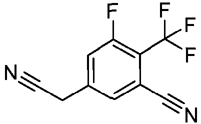 | 0.34 | 0.54 | 0.56 | 1.44 |
| 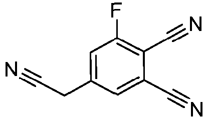 | 0.34 | 0.66 | 0.56 | 1.56 |
| 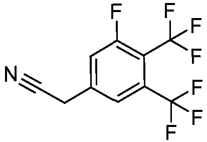 | 0.34 | 0.54 | 0.43 | 1.31 |
| 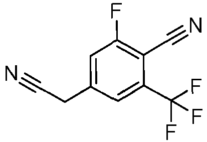 | 0.34 | 0.66 | 0.43 | 1.43 |
| 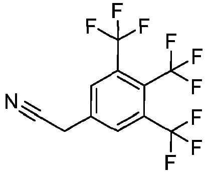 | 0.43 | 0.54 | 0.43 | 1.40 |
| 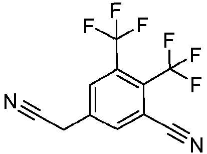 | 0.43 | 0.54 | 0.56 | 1.53 |
| 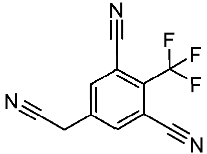 | 0.56 | 0.54 | 0.56 | 1.66 |
| 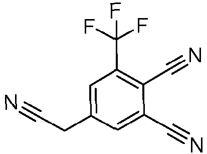 | 0.43 | 0.66 | 0.56 | 1.65 |
| 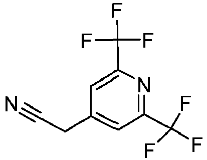 | 0.43 | - | 0.43 | 0.86 |
| 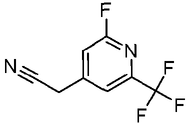 | 0.34 | - | 0.43 | 0.77 |
| 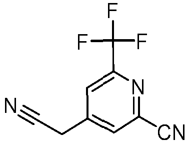 | 0.43 | - | 0.56 | 0.99 |
| 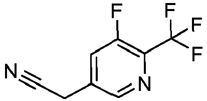 | 0.34 | 0.54 | - | 0.88 |
| 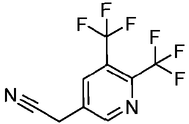 | 0.43 | 0.54 | - | 0.97 |
| 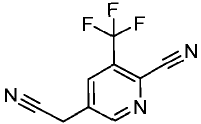 | 0.54 | 0.66 | - | 1.20 |
| 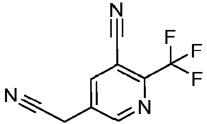 | 0.56 | 0.54 | - | 1.10 |
| 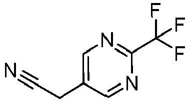 | - | 0.54 | - | 0.54 |
| 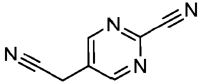 | - | 0.66 | - | 0.66 |
| 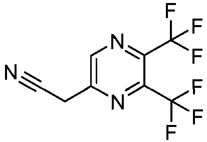 | - | 0.54 | 0.43 | 0.97 |
| 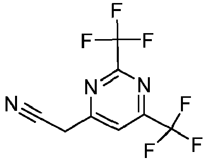 | 0.43 | - | 0.43 | 0.86 |
| 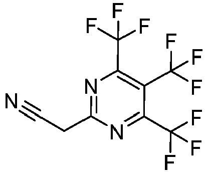 | 0.43 | 0.54 | 0.43 | 1.40 |

According to one embodiment of the present invention, formula (III) is selected from the group comprising the following moieties: 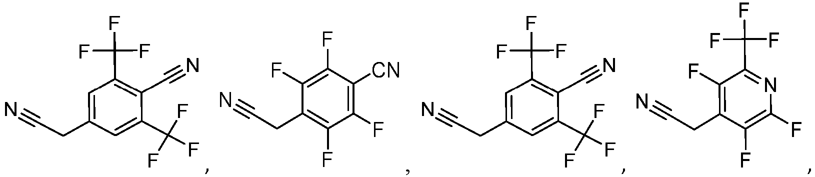 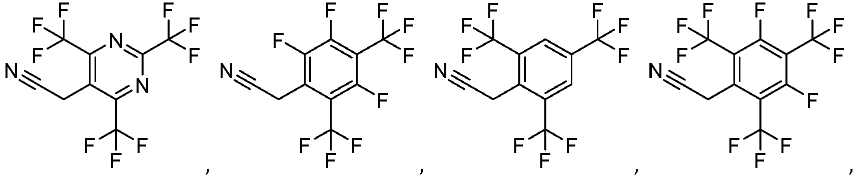 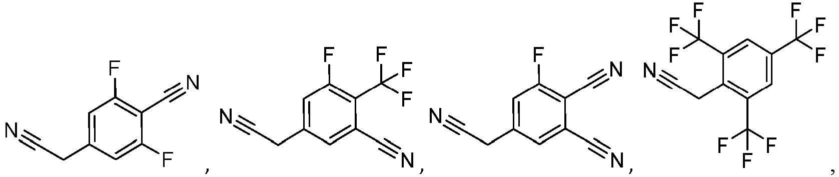 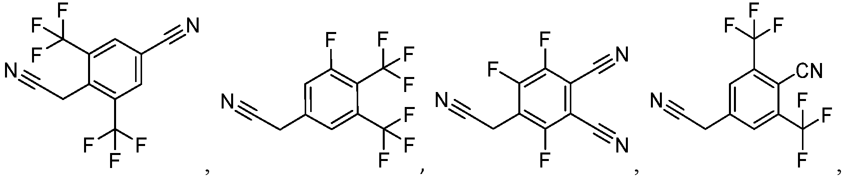 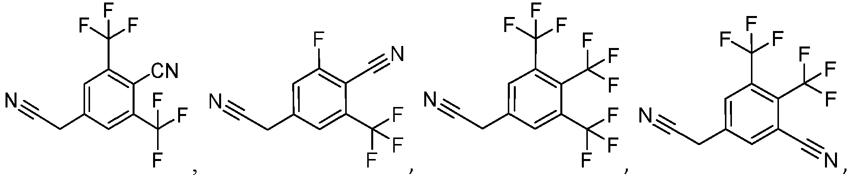 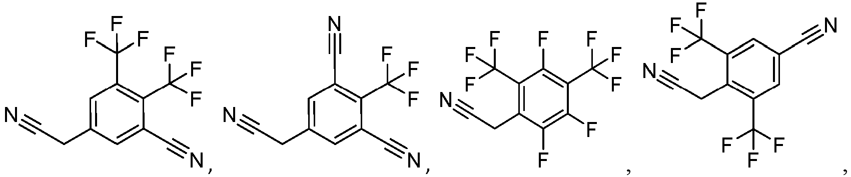 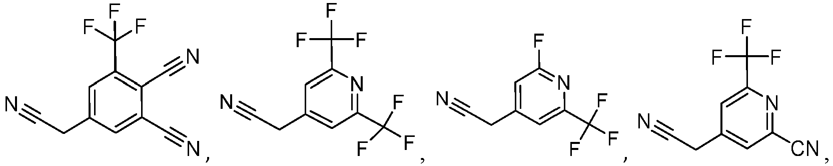 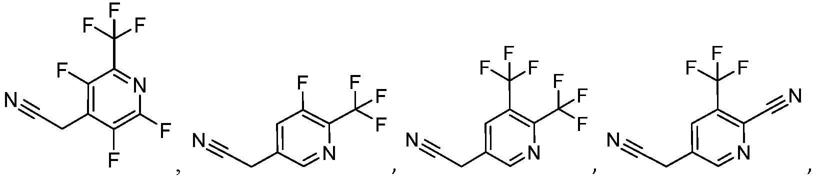 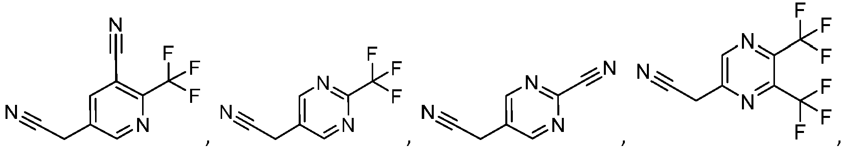 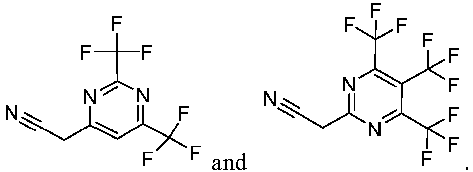
According to one embodiment, compound of formula (I) is selected from the compounds A1 to A82:

| | A¹ | A² | A³ |
|---|---|---|---|
| A1 | 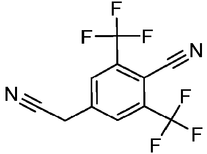 | 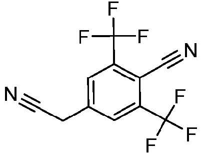 | 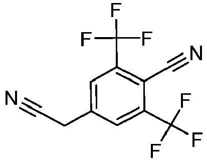 |
| A2 | 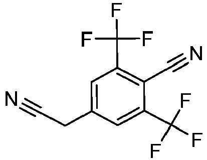 | 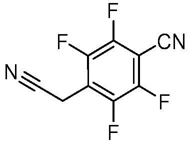 | 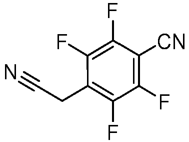 |
| A3 | 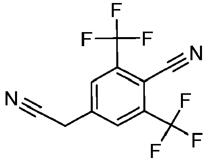 | 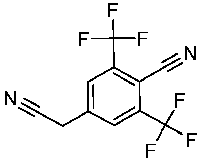 | 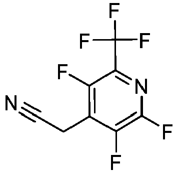 |
| A4 | 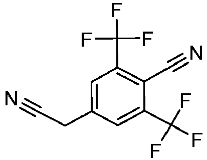 | 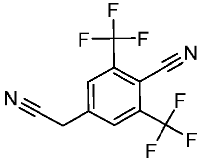 | 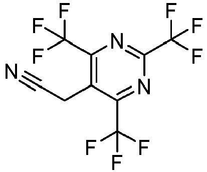 |
| A5 | 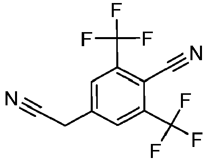 | 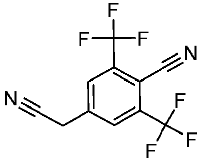 | 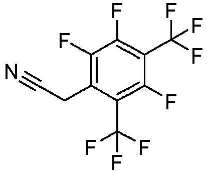 |
| A6 | 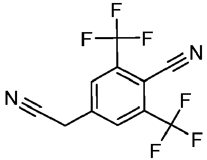 | 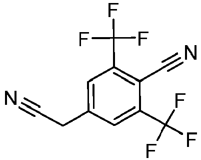 | 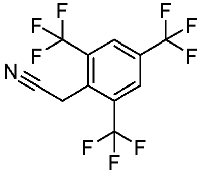 |
| A7 | 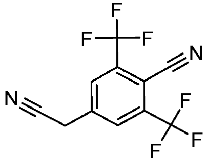 | 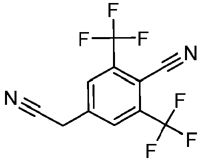 | 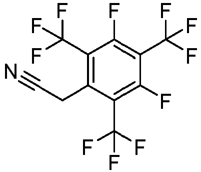 |
| A8 | 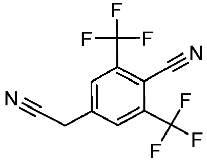 | 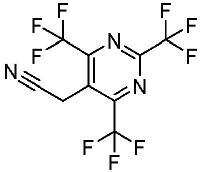 | 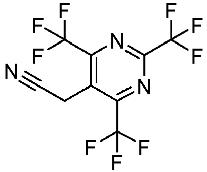 |
| A9 | 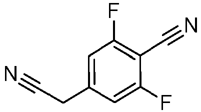 | 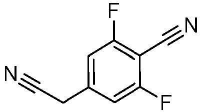 | 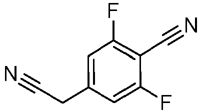 |
| A10 | 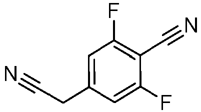 | 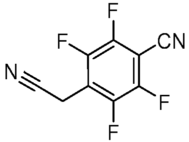 | 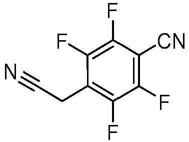 |
| A11 | 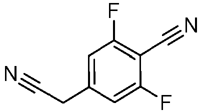 | 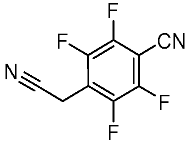 | 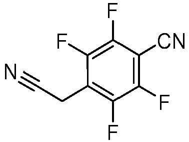 |
| A12 | 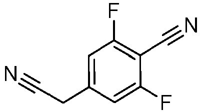 | 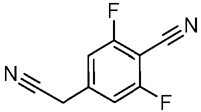 | 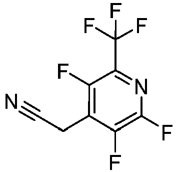 |
| A13 | 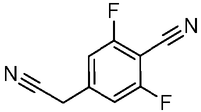 | 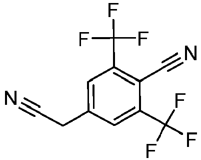 | 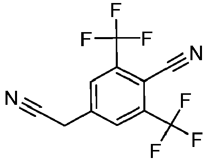 |
| A14 | 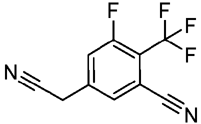 | 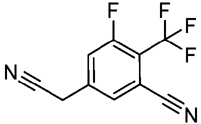 | 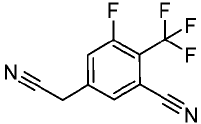 |
| A15 | 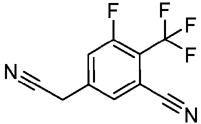 | 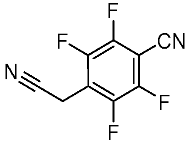 | 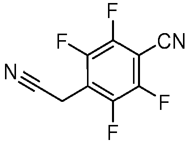 |
| A16 | 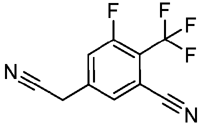 | 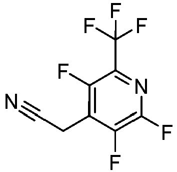 | 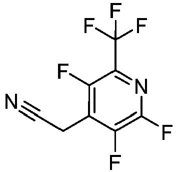 |
| A17 | 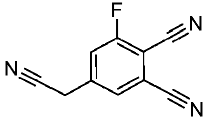 | 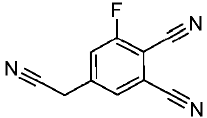 | 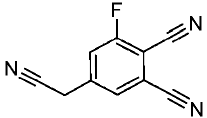 |
| A18 | 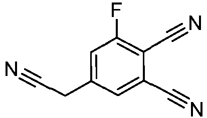 | 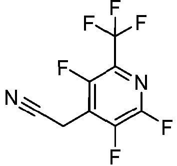 | 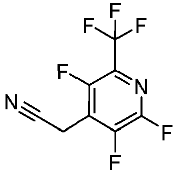 |
| A19 | 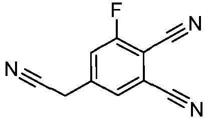 | 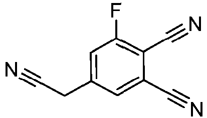 | 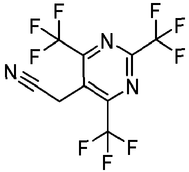 |
| A20 | 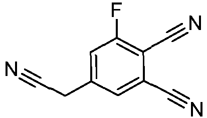 | 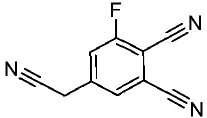 | 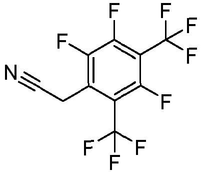 |
| A21 | 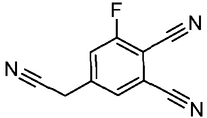 | 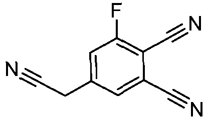 | 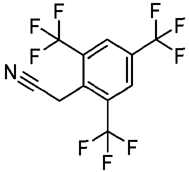 |
| A22 | 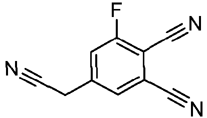 | 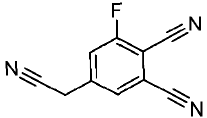 | 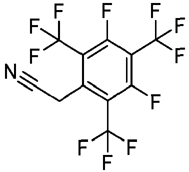 |
| A23 | 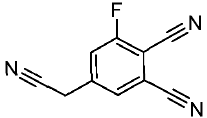 | 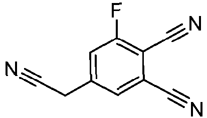 | 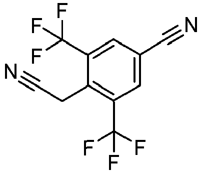 |
| A24 | 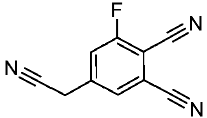 | 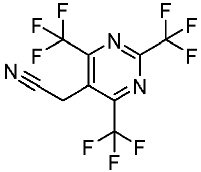 | 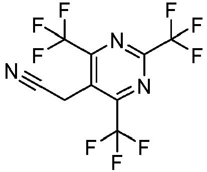 |
| A25 | 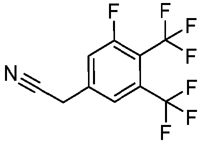 | 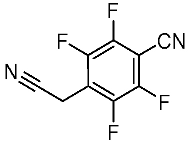 | 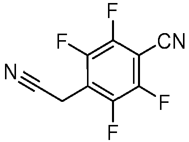 |
| A26 | 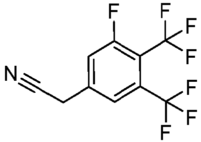 | 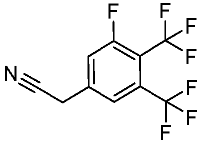 | 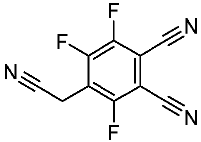 |
| A27 | 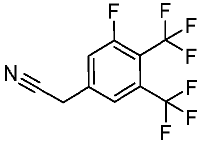 | 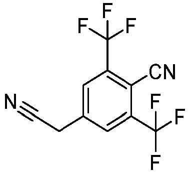 | 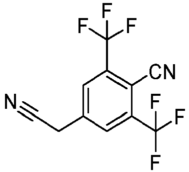 |
| A28 | 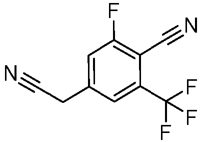 | 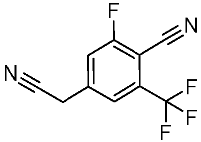 | 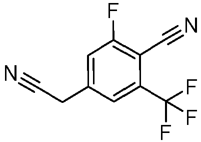 |
| A29 | 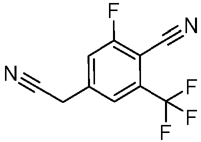 | 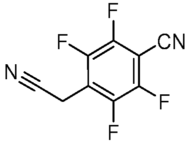 | 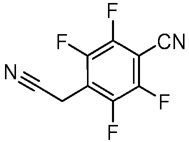 |
| A30 | 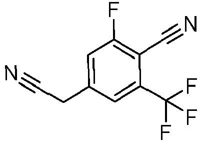 | 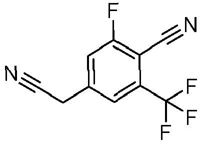 | 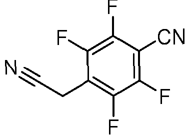 |
| A31 | 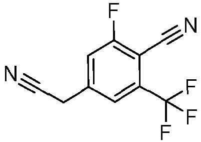 | 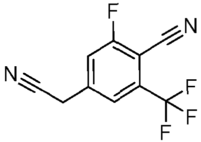 | 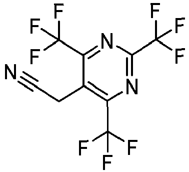 |
| A32 | 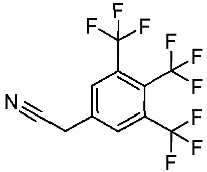 | 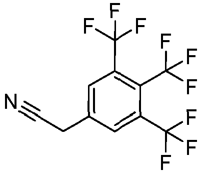 | 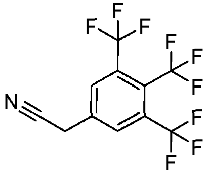 |
| A33 | 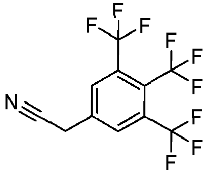 | 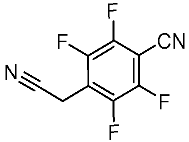 | 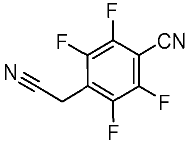 |
| A34 | 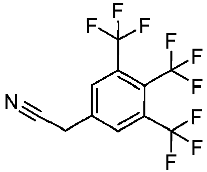 | 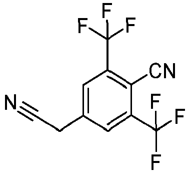 | 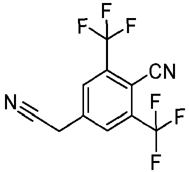 |
| A35 | 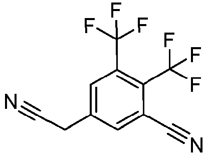 | 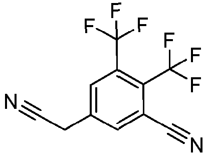 | 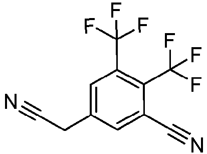 |
| A36 | 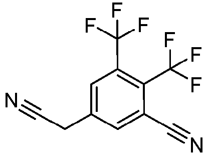 | 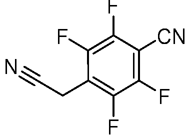 | 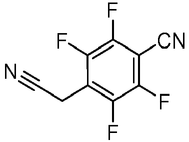 |
| A37 | 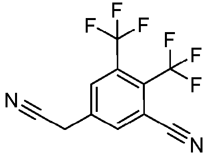 | 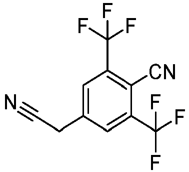 | 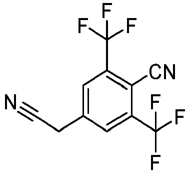 |
| A38 | 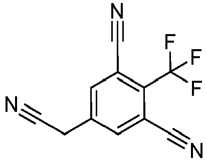 | 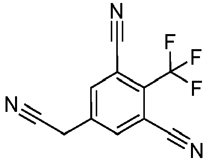 | 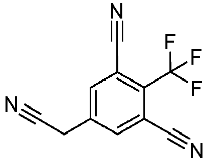 |
| A39 | 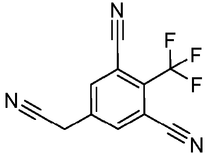 | 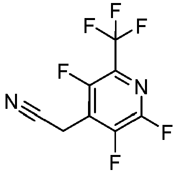 | 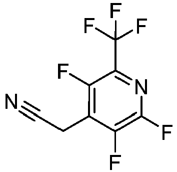 |
| A40 | 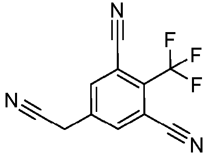 | 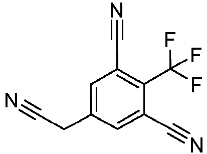 | 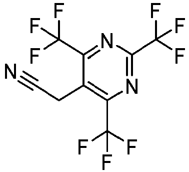 |
| A41 | 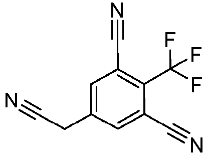 | 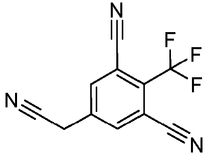 | 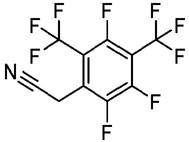 |
| A42 | 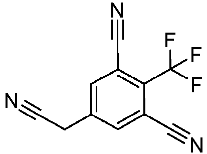 | 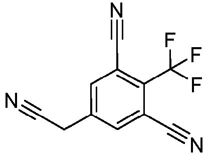 | 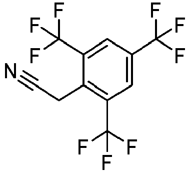 |
| A43 | 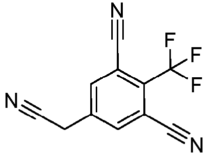 | 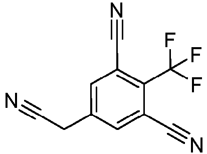 | 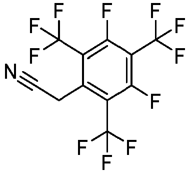 |
| A44 | 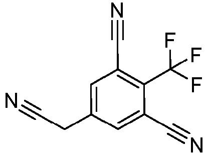 | 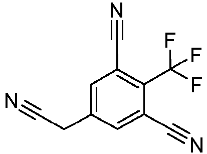 | 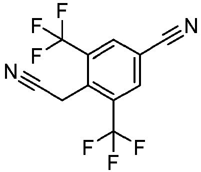 |
| A45 | 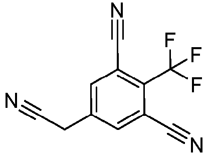 | 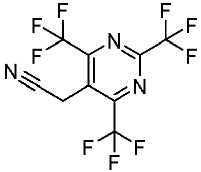 | 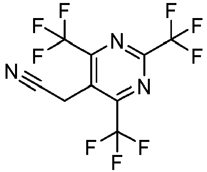 |
| A46 | 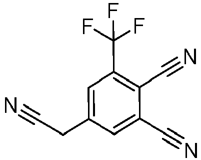 | 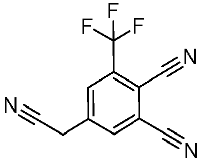 | 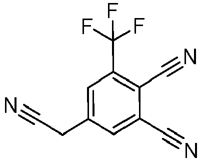 |
| A47 | 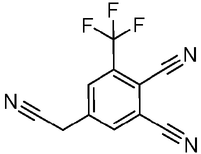 | 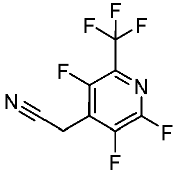 | 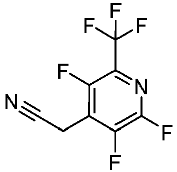 |
| A48 | 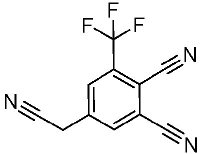 | 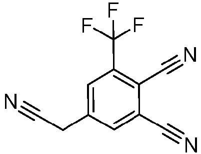 | 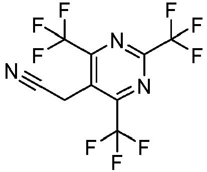 |
| A49 | 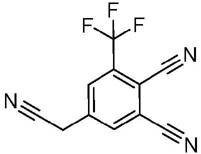 | 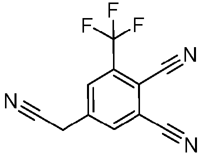 | 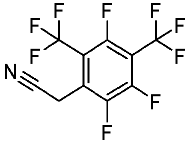 |
| A50 | 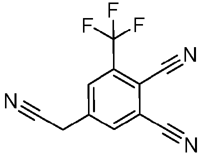 | 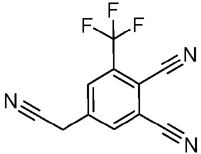 | 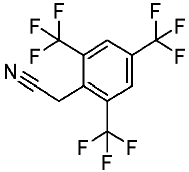 |
| A51 | 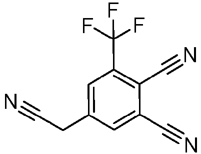 | 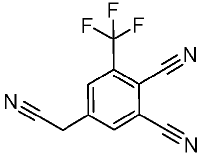 | 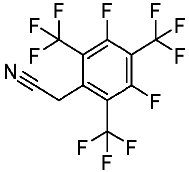 |
| A52 | 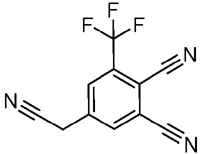 | 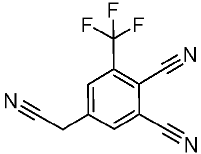 | 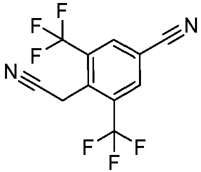 |
| A53 | 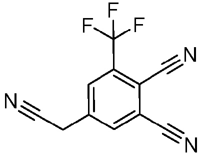 | 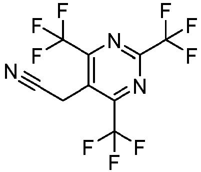 | 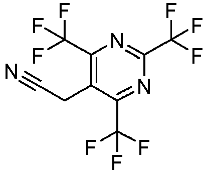 |
| A54 | 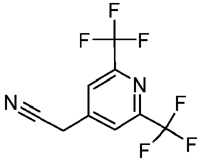 | 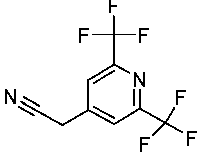 | 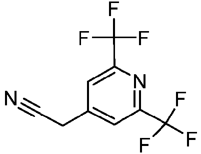 |
| A55 | 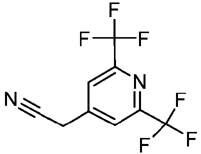 | 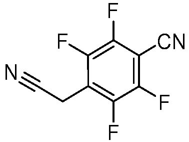 | 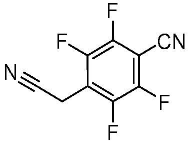 |
| A56 | 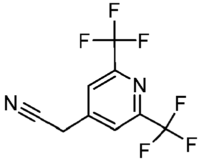 | 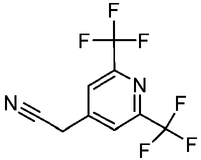 | 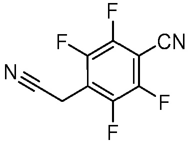 |
| A57 | 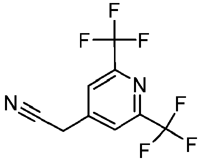 | 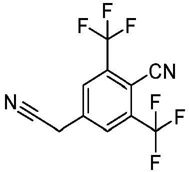 | 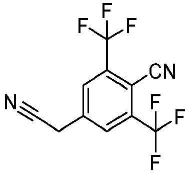 |
| A58 | 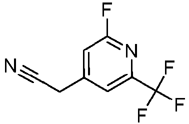 | 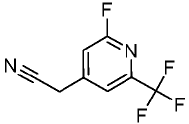 | 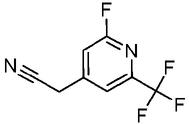 |
| A59 | 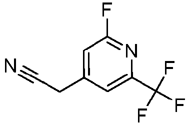 | 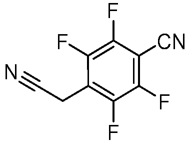 | 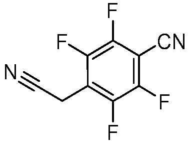 |
| A60 | 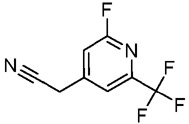 | 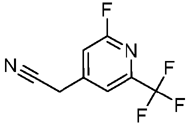 | 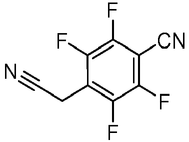 |
| A61 | 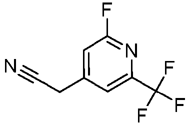 | 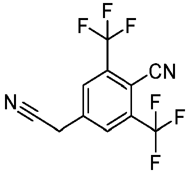 | 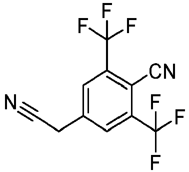 |
| A62 | 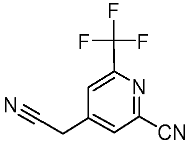 | 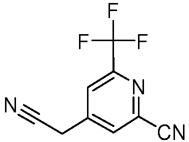 | 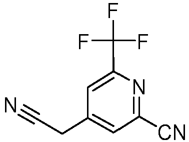 |
| A63 | 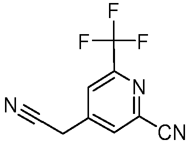 | 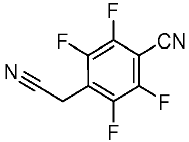 | 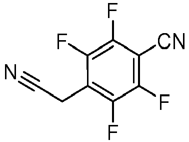 |
| A64 | 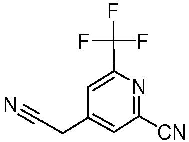 | 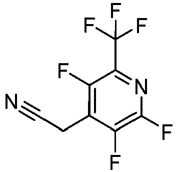 | 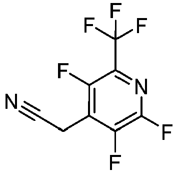 |
| A65 | 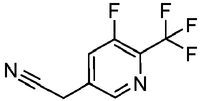 | 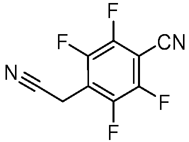 | 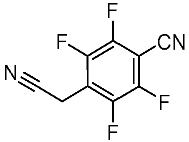 |
| A66 | 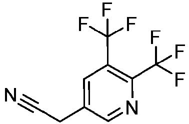 | 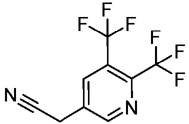 | 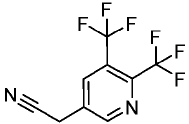 |
| A67 | 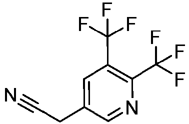 | 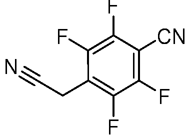 | 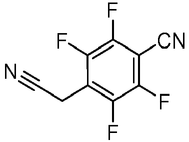 |
| A68 | 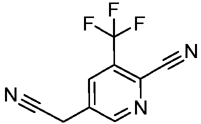 | 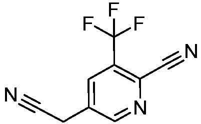 | 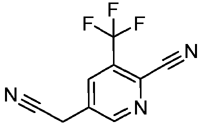 |
| A69 | 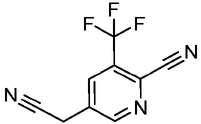 | 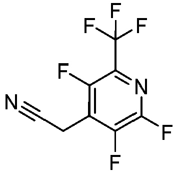 | 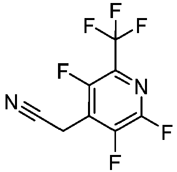 |
| A70 | 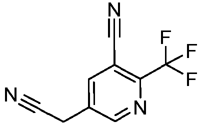 | 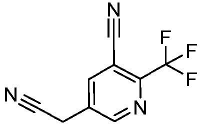 | 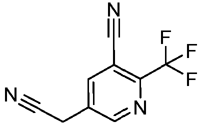 |
| A71 | 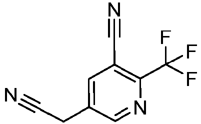 | 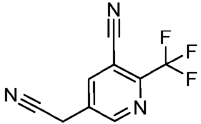 | 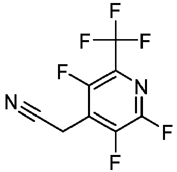 |
| A72 | 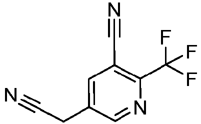 | 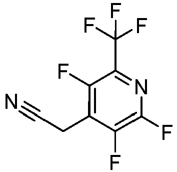 | 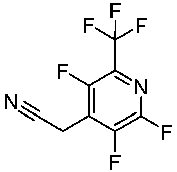 |
| A73 | 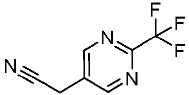 | 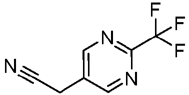 | 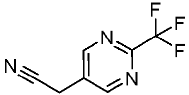 |
| A74 | 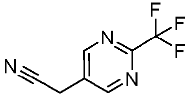 | 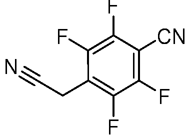 | 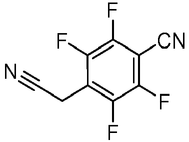 |
| A75 | 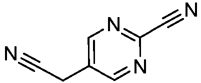 | 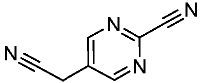 | 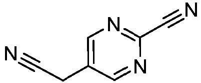 |
| A76 | 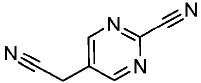 | 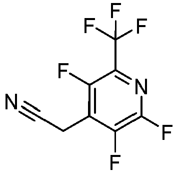 | 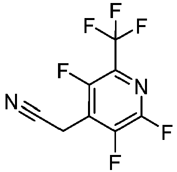 |
| A77 | 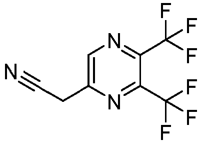 | 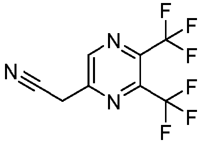 | 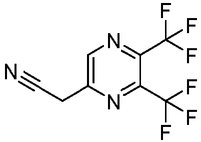 |
| A78 | 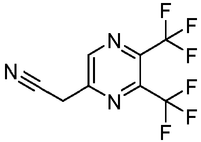 | 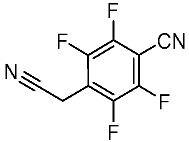 | 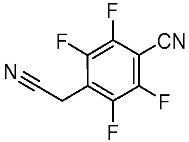 |
| A79 | 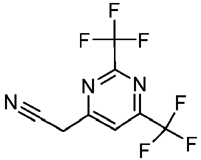 | 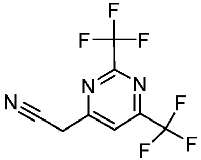 | 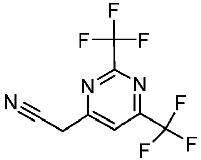 |
| A80 | 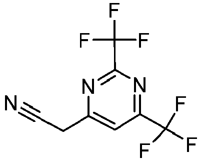 | 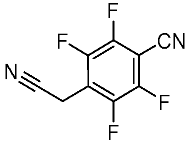 | 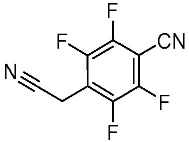 |
| A81 | 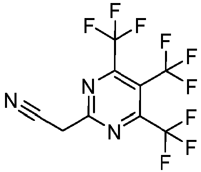 | 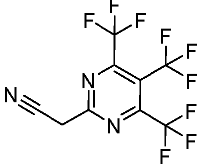 | 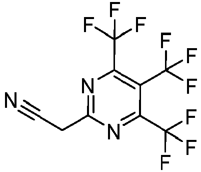 |
| A82 | 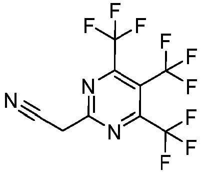 | 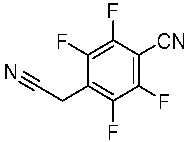 | 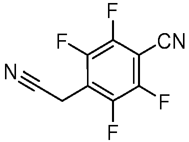 |

The present invention furthermore relates to compound of formula (I) of claim 1 having less than nine cyano moieties and a LUMO of ≤ -5.05 eV.

According to one embodiment of the present invention, the compound of formula (I) comprises between 3 and 8 cyano moieties and a LUMO of ≤ -5.05 eV.

According to one embodiment of the present invention, the compound of formula (I) comprises between 6 and 8 cyano moieties and a LUMO of ≤ -5.05 eV.

According to one embodiment of the present invention the p-type and/or n-type charge generation layer and/or the compound of formula (I) are non-emissive.

In the context of the present specification the term "essentially non-emissive" or "non-emissive" means that the contribution of the compound or layer to the visible emission spectrum from the device is less than 10 %, preferably less than 5 % relative to the visible emission spectrum. The visible emission spectrum is an emission spectrum with a wavelength of about ≥ 380 nm to about ≤ 780 nm.

According to one embodiment of the invention, the p-type charge generation layer is arranged closer to the cathode layer than the n-type charge generation layer.

According to one embodiment of the invention, the p-type charge generation layer further comprises a substantially covalent matrix compound.

### Substantially covalent matrix compound

The organic semiconductor layer may further comprises a substantially covalent matrix compound. According to one embodiment the substantially covalent matrix compound may be selected from at least one organic compound. The substantially covalent matrix may consists substantially from covalently bound C, H, O, N, S, which optionally comprise in addition covalently bound B, P, As and/or Se.

According to one embodiment of the organic electronic device, the organic semiconductor layer further comprises a substantially covalent matrix compound, wherein the substantially covalent matrix compound may be selected from organic compounds consisting substantially from covalently bound C, H, O, N, S, which optionally comprise in addition covalently bound B, P, As and/or Se.

Organometallic compounds comprising covalent bonds carbon-metal, metal complexes comprising organic ligands and metal salts of organic acids are further examples of organic compounds that may serve as substantially covalent matrix compounds of the hole injection layer.

In one embodiment, the substantially covalent matrix compound lacks metal atoms and majority of its skeletal atoms may be selected from C, O, S, N. Alternatively, the substantially covalent matrix compound lacks metal atoms and majority of its skeletal atoms may be selected from C and N.

According to one embodiment, the substantially covalent matrix compound may have a molecular weight Mw of ≥ 400 and < 2000 g/mol, preferably a molecular weight Mw of ≥ 450 and ≤ 1500 g/mol, further preferred a molecular weight Mw of ≥ 500 and ≤ 1000 g/mol, in addition preferred a molecular weight Mw of ≥ 550 and ≤ 900 g/mol, also preferred a molecular weight Mw of ≥ 600 and ≤ 800 g/mol.

Preferably, the substantially covalent matrix compound comprises at least one arylamine moiety, alternatively a diarylamine moiety, alternatively a triarylamine moiety.

Preferably, the substantially covalent matrix compound is free of metals and/or ionic bonds.

### Compound of formula (VI) or a compound of formula (VII)

According to another aspect of the present invention, the at least one matrix compound, also referred to as "substantially covalent matrix compound", may comprises at least one arylamine compound, diarylamine compound, triarylamine compound, a compound of formula (VI) or a compound of formula (VII): 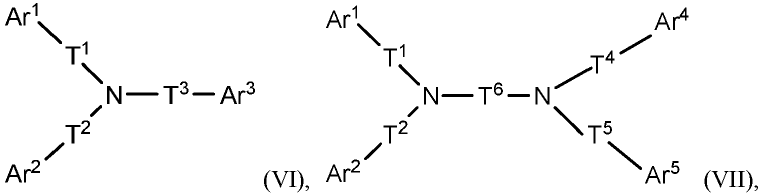 wherein:
T¹, T², T³, T⁴ and T⁵ are independently selected from a single bond, phenylene, biphenylene, terphenylene or naphthenylene, preferably a single bond or phenylene;
T⁶ is phenylene, biphenylene, terphenylene or naphthenylene;
Ar¹, Ar², Ar³, Ar⁴ and Ar⁵ are independently selected from substituted or unsubstituted C₆ to C₂₀ aryl, or substituted or unsubstituted C₃ to C₂₀ heteroarylene, substituted or unsubstituted biphenylene, substituted or unsubstituted fluorene, substituted 9-fluorene, substituted 9,9-fluorene, substituted or unsubstituted naphthalene, substituted or unsubstituted anthracene, substituted or unsubstituted phenanthrene, substituted or unsubstituted pyrene, substituted or unsubstituted perylene, substituted or unsubstituted triphenylene, substituted or unsubstituted tetracene, substituted or unsubstituted tetraphene, substituted or unsubstituted dibenzofurane, substituted or unsubstituted dibenzothiophene, substituted or unsubstituted xanthene, substituted or unsubstituted carbazole, substituted 9-phenylcarbazole, substituted or unsubstituted azepine, substituted or unsubstituted dibenzo[b,f]azepine, substituted or unsubstituted 9,9'-spirobi[fluorene], substituted or unsubstituted spiro[fluorene-9,9'-xanthene], or a substituted or unsubstituted aromatic fused ring system comprising at least three substituted or unsubstituted aromatic rings selected from the group comprising substituted or unsubstituted non-hetero, substituted or unsubstituted hetero 5-member rings, substituted or unsubstituted 6-member rings and/or substituted or unsubstituted 7-member rings, substituted or unsubstituted fluorene, or a fused ring system comprising 2 to 6 substituted or unsubstituted 5- to 7-member rings and the rings are selected from the group comprising (i) unsaturated 5- to 7-member ring of a heterocycle, (ii) 5- to 6-member of an aromatic heterocycle, (iii) unsaturated 5- to 7-member ring of a non-heterocycle, (iv) 6-member ring of an aromatic non-heterocycle;
wherein
the substituents of Ar¹, Ar², Ar³, Ar⁴ and Ar⁵ are selected the same or different from the group comprising H, D, F, C(-O)R², CN, Si(R²)₃, P(-O)(R²)₂, OR², S(-O)R², S(-O)₂R², substituted or unsubstituted straight-chain alkyl having 1 to 20 carbon atoms, substituted or unsubstituted branched alkyl having 1 to 20 carbon atoms, substituted or unsubstituted cyclic alkyl having 3 to 20 carbon atoms, substituted or unsubstituted alkenyl or alkynyl groups having 2 to 20 carbon atoms, substituted or unsubstituted alkoxy groups having 1 to 20 carbon atoms, substituted or unsubstituted aromatic ring systems having 6 to 40 aromatic ring atoms, and substituted or unsubstituted heteroaromatic ring systems having 5 to 40 aromatic ring atoms, unsubstituted C₆ to C₁₈ aryl, unsubstituted C₃ to C₁₈ heteroaryl, a fused ring system comprising 2 to 6 unsubstituted 5- to 7-member rings and the rings are selected from the group comprising unsaturated 5- to 7-member ring of a heterocycle, 5- to 6-member of an aromatic heterocycle, unsaturated 5- to 7-member ring of a non-heterocycle, and 6-member ring of an aromatic non-heterocycle,
wherein R² may be selected from H, D, straight-chain alkyl having 1 to 6 carbon atoms, branched alkyl having 1 to 6 carbon atoms, cyclic alkyl having 3 to 6 carbon atoms, alkenyl or alkynyl groups having 2 to 6 carbon atoms, C₆ to C₁₈ aryl or C₃ to C₁₈ heteroaryl.

According to an embodiment wherein T¹, T², T³, T⁴ and T⁵ may be independently selected from a single bond, phenylene, biphenylene or terphenylene. According to an embodiment wherein T¹, T², T³, T⁴ and T⁵ may be independently selected from phenylene, biphenylene or terphenylene and one of T¹, T², T³, T⁴ and T⁵ are a single bond. According to an embodiment wherein T¹, T², T³, T⁴ and T⁵ may be independently selected from phenylene or biphenylene and one of T¹, T², T³, T⁴ and T⁵ are a single bond. According to an embodiment wherein T¹, T², T³, T⁴ and T⁵ may be independently selected from phenylene or biphenylene and two of T¹, T², T³, T⁴ and T⁵ are a single bond.

According to an embodiment wherein T¹, T² and T³ may be independently selected from phenylene and one of T¹, T² and T³ are a single bond. According to an embodiment wherein T¹, T² and T³ may be independently selected from phenylene and two of T¹, T² and T³ are a single bond.

According to an embodiment wherein T⁶ may be phenylene, biphenylene, terphenylene. According to an embodiment wherein T⁶ may be phenylene. According to an embodiment wherein T⁶ may be biphenylene. According to an embodiment wherein T⁶ may be terphenylene.

According to an embodiment wherein Ar¹, Ar², Ar³, Ar⁴ and Ar⁵ may be independently selected from D1 to D16: 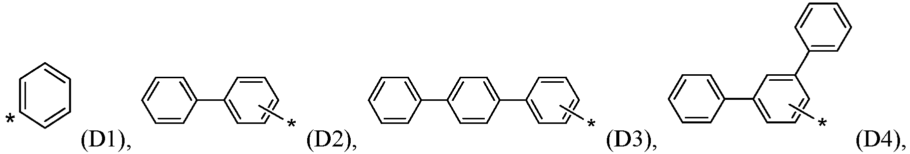 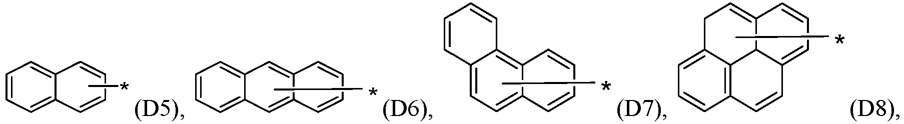 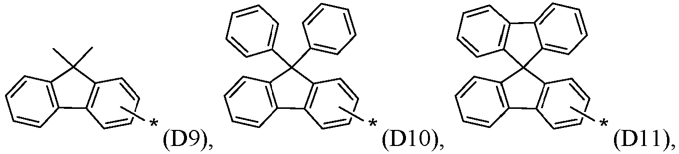 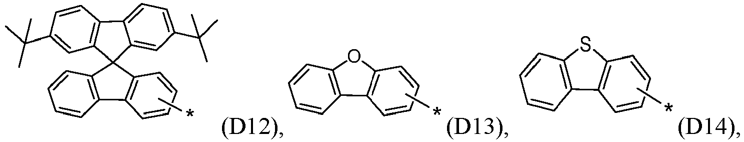 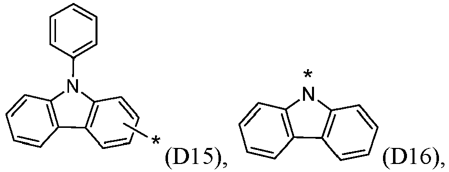 wherein the asterix "*" denotes the binding position.

According to an embodiment, wherein Ar¹, Ar², Ar³, Ar⁴ and Ar⁵ may be independently selected from D1 to D15; alternatively selected from D1 to D10 and D13 to D15.

According to an embodiment, wherein Ar¹, Ar², Ar³, Ar⁴ and Ar⁵ may be independently selected from the group consisting of D1, D2, D5, D7, D9, D10, D13 to D16.

The rate onset temperature may be in a range particularly suited to mass production, when Ar¹, Ar², Ar³, Ar⁴ and Ar⁵ are selected in this range.

The "matrix compound of formula (VI) or formula (VII)" may be also referred to as "hole transport compound".

According to one embodiment, the substantially covalent matrix compound comprises at least one naphthyl group, carbazole group, dibenzofuran group, dibenzothiophene group and/or substituted fluorenyl group, wherein the substituents are independently selected from methyl, phenyl or fluorenyl.

According to an embodiment of the electronic device, wherein the matrix compound of formula (VI) or formula (VII) are selected from F1 to F18:

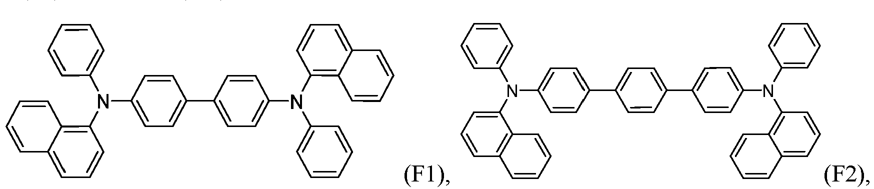

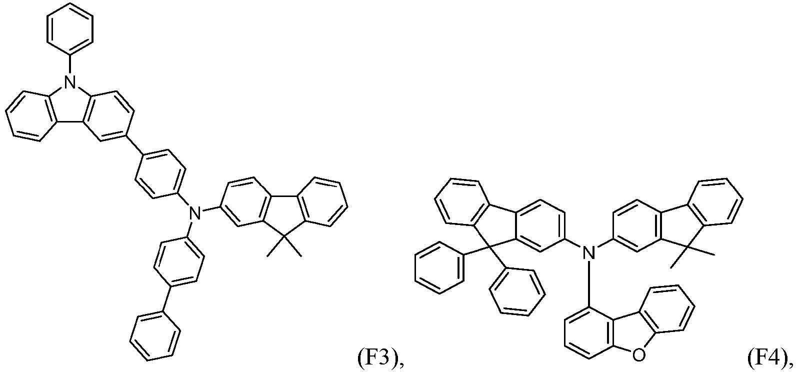

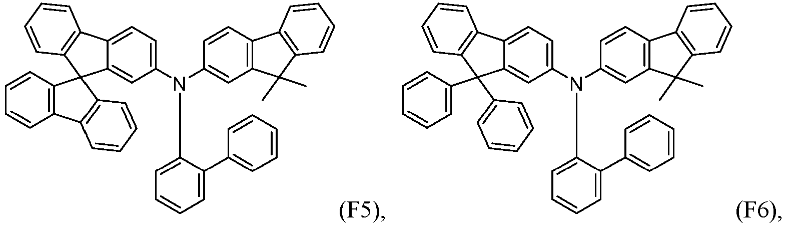

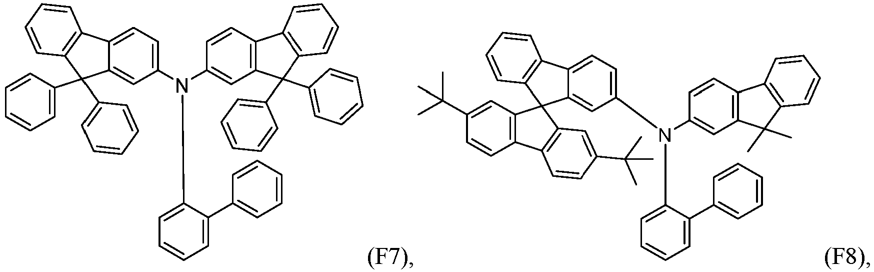

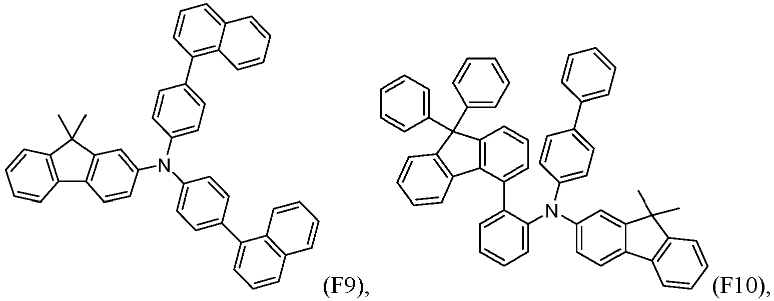

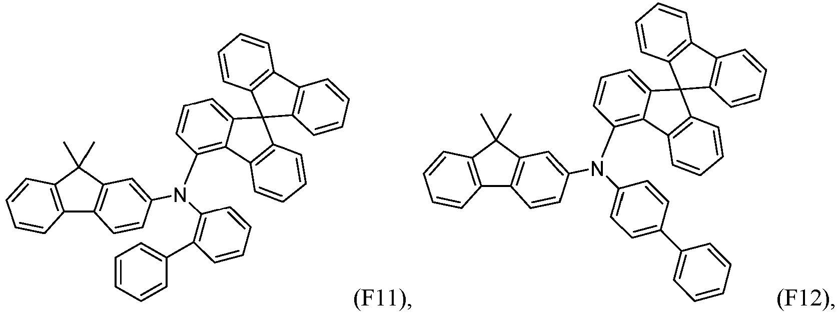

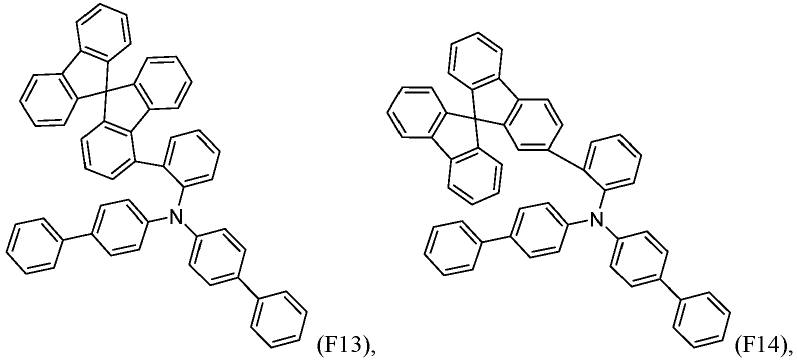

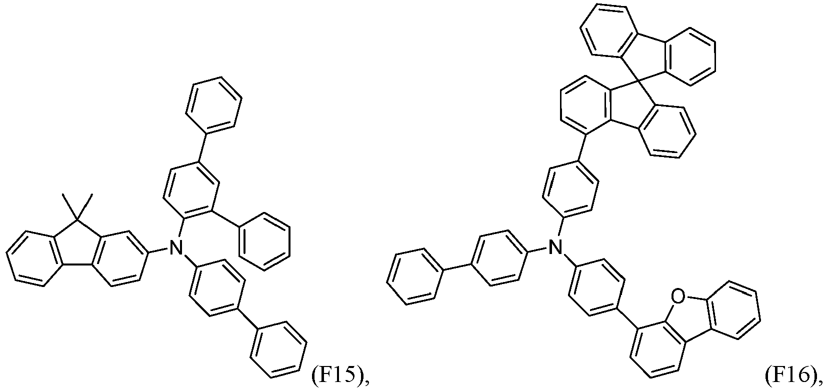

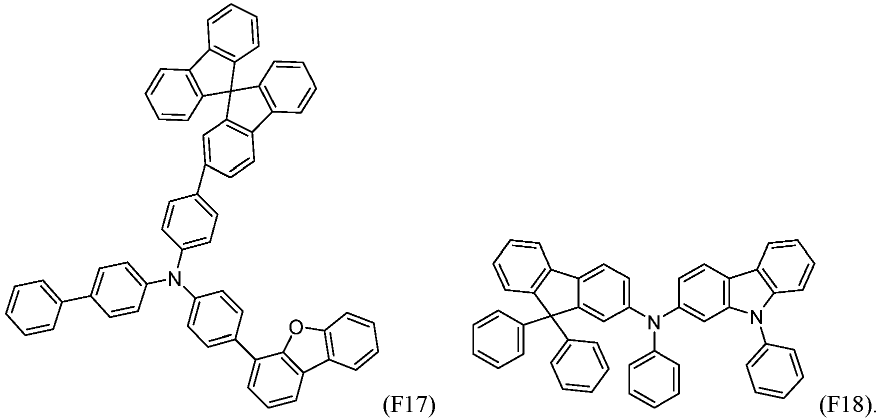

According to one embodiment of the invention the electronic organic device is an electroluminescent device, preferably an organic light emitting diode.

According to a preferred embodiment of the invention, the electronic organic device is an electroluminescent device, preferably an organic light emitting diode, wherein light is emitted through the cathode layer.

The present invention furthermore relates to a display device comprising an organic electronic device according to the present invention.

According to a preferred embodiment of the invention, the display device comprising an organic electronic device according to the present invention, wherein the cathode layer is transparent.

### p-type charge generation layer

The p-type charge generation layer may be formed on the anode layer or cathode layer by vacuum deposition, spin coating, printing, casting, slot-die coating, Langmuir-Blodgett (LB) deposition, or the like. When the a p-type charge generation layer is formed using vacuum deposition, the deposition conditions may vary according to the compound(s) that are used to form the layer, and the desired structure and thermal properties of the layer. In general, however, conditions for vacuum deposition may include a deposition temperature of 100° C to 350° C, a pressure of 10⁻⁸ to 10⁻³ Torr (1 Torr equals 133.322 Pa), and a deposition rate of 0.1 to 10 nm/sec.

When the a p-type charge generation layer is formed using spin coating or printing, coating conditions may vary according to the compound(s) that are used to form the layer, and the desired structure and thermal properties of the organic semiconductor layer. For example, the coating conditions may include a coating speed of about 2000 rpm to about 5000 rpm, and a thermal treatment temperature of about 80° C to about 200° C. Thermal treatment removes a solvent after the coating is performed.

The thickness of the p-type charge generation layer may be in the range from about 1 nm to about 20 nm, and for example, from about 2 nm to about 15 nm, alternatively about 2 nm to about 12 nm.

According to one embodiment of the present invention, the p-type charge generation layer may comprise:
- at least about ≥ 0.5 wt.-% to about ≤ 30 wt.-%, preferably about ≥ 0.5 wt.-% to about ≤ 20 wt.-%, and more preferred about ≥ 1 wt.-% to about ≤ 15 wt.-% of a compound of formula (I), and
- at least about ≥ 70 wt.-% to about ≤ 99.5 wt.-%, preferably about ≥ 80 wt.-% to about ≤ 99.5 wt.-%, and more preferred about ≥ 85 wt.-% to about ≤ 99 wt.-% of a substantially covalent matrix compound; preferably the wt.-% of the compound of formula (I) is lower than the wt.-% of the substantially covalent matrix compound; wherein the weight-% of the components are based on the total weight of the p-type charge generation layer.

According to one embodiment of the present invention, the p-type charge generation layer may comprise:
- at least about ≥ 0.5 vol.-% to about ≤ 30 vol.-%, preferably about ≥ 0.5 vol.-% to about ≤ 20 vol.-%, and more preferred about ≥ 1 vol.-% to about ≤ 15 vol.-% of a compound of formula (I), and
- at least about ≥ 70 vol.-% to about ≤ 99.5 vol.-%, preferably about ≥ 80 vol.-% to about ≤ 99.5 vol.-%, and more preferred about ≥ 85 vol.-% to about ≤ 99 vol.-% of a substantially covalent matrix compound; preferably the vol.-% of the compound of formula (I) is lower than the vol.-% of the substantially covalent matrix compound; wherein the weight-% of the components are based on the total weight of the p-type charge generation layer.

### n-type charge generation layer

As indicated, the charge generation layer may additionally comprise a n-type charge generation layer.

According to an embodiment of the present invention, the n-type charge generation layer may comprise an n-CGL matrix compound, preferably comprising at least one C₂ to C₂₄ N-heteroaryl or P=X group, with X being O, P, Se, with P=O especially preferred.

According to an embodiment of the present invention, the at least one C₂ to C₂₄ N-heteroaryl may be selected from a compound comprising at least one azine group, preferably at least two azine groups, also preferred three azine groups.

According to an embodiment of the present invention, the n-type charge generation layer may comprise an n-CGL matrix compound comprising at least one group selected from the list consisting of pyridine, pyrimidine, triazine, imidazole, benzimidazole, benzooxazole, quinone, benzoquinone, quinoxaline, benzoquinoxaline, acridine, phenanthroline, benzoacridine, dibenzoacridine.

According to an embodiment of the present invention, the n-type charge generation layer may comprise an n-CGL matrix compound comprising at least one phenanthroline group, preferably two phenanthroline groups.

According to an embodiment of the present invention, the n-type charge generation layer may comprise metal a dopant, wherein the metal dopant may be a metal selected from Li, Na, Cs, Mg, Ca, Sr, S or Yb, preferably from Li or Yb.

### Further layers

In accordance with the invention, the organic electronic device may comprise, besides the layers already mentioned above, further layers. Exemplary embodiments of respective layers are described in the following:

### Substrate

The substrate may be any substrate that is commonly used in manufacturing of, electronic devices, such as organic light-emitting diodes. If light is to be emitted through the substrate, the substrate shall be a transparent or semitransparent material, for example a glass substrate or a transparent plastic substrate. If light is to be emitted through the top surface, the substrate may be both a transparent as well as a non-transparent material, for example a glass substrate, a plastic substrate, a metal substrate, a silicon substrate or a backplane.

### Anode layer

The anode layer may be formed by depositing or sputtering a material that is used to form the anode layer. The material used to form the anode layer may be a high work-function material, so as to facilitate hole injection. The anode material may also be selected from a low work function material (i.e. aluminum). The anode electrode may be a transparent or reflective electrode. Transparent conductive oxides, such as indium tin oxide (ITO), indium zinc oxide (IZO), tin-dioxide (SnO2), aluminum zinc oxide (AlZO) and zinc oxide (ZnO), may be used to form the anode electrode. The anode layer may also be formed using metals, typically silver (Ag), gold (Au), or metal alloys.

### Hole injection layer

A hole injection layer (HIL) may be formed on the anode layer by vacuum deposition, spin coating, printing, casting, slot-die coating, Langmuir-Blodgett (LB) deposition, or the like. When the HIL is formed using vacuum deposition, the deposition conditions may vary according to the compound that is used to form the HIL, and the desired structure and thermal properties of the HIL. In general, however, conditions for vacuum deposition may include a deposition temperature of 100° C to 500° C, a pressure of 10⁻⁸ to 10⁻³ Torr (1 Torr equals 133.322 Pa), and a deposition rate of 0.1 to 10 nm/sec.

When the HIL is formed using spin coating or printing, coating conditions may vary according to the compound that is used to form the HIL, and the desired structure and thermal properties of the HIL. For example, the coating conditions may include a coating speed of about 2000 rpm to about 5000 rpm, and a thermal treatment temperature of about 80° C to about 200° C. Thermal treatment removes a solvent after the coating is performed.

The HIL may be formed of any compound that is commonly used to form a HIL. Examples of compounds that may be used to form the HIL include a phthalocyanine compound, such as copper phthalocyanine (CuPc), 4,4',4"-tris (3-methylphenylphenylamino) triphenylamine (m-MTDATA), TDATA, 2T-NATA, polyaniline/dodecylbenzenesulfonic acid (Pani/DBSA), poly(3,4-ethylenedioxythiophene)/poly(4-styrenesulfonate) (PEDOT/PSS), polyaniline/camphor sulfonic acid (Pani/CSA), and polyaniline)/poly(4-styrenesulfonate (PANI/PSS).

The HIL may comprise or consist of p-type dopant and the p-type dopant may be selected from tetrafluoro-tetracyanoquinonedimethane (F4TCNQ), 2,2'-(perfluoronaphthalen-2,6-diylidene) dimalononitrile or 2,2',2"-(cyclopropane-1,2,3-triylidene)tris(2-(p-cyanotetrafluorophenyl)acetonitrile) but not limited hereto.

Preferably, the p-type dopant is selected from a radialene compound, for example 2,2',2"-(cyclopropane-1,2,3-triylidene)tris(2-(p-cyanotetrafluorophenyl)acetonitrile) (CC3).

The HIL may comprise a substantially covalent matrix compound and a p-type dopant.

The p-type dopant concentrations can be selected from 1 to 20 wt.-%, more preferably from 3 wt.-% to 10 wt.-%.

The p-type dopant concentrations can be selected from 1 to 20 vol.-%, more preferably from 3 vol.-% to 10 vol.-%.

According to a preferred embodiment of the present invention, however, the HIL comprises a compound of formula (I) or (IV) as described above.

According to a preferred embodiment of the present invention, the HIL may comprises the same compound of formula (I) and/or (IV) as in the p-type charge generation layer. According to a preferred embodiment of the present invention, the HIL may comprises a substantially covalent matrix compound as described above.

According to a preferred embodiment of the present invention, the HIL may comprises a compound of formula (I) or (IV), as described above, and a compound of formula (VI) or (VII), as described above.

According to a preferred embodiment of the present invention, the p-type charge generation layer and the hole injection layer may comprise an identical substantially covalent matrix compound.

The thickness of the HIL may be in the range from about 1 nm to about 100 nm, and for example, from about 1 nm to about 25 nm. When the thickness of the HIL is within this range, the HIL may have excellent hole injecting characteristics, without a substantial penalty in driving voltage.

### Hole transport layer

A hole transport layer (HTL) may be formed on the HIL by vacuum deposition, spin coating, slot-die coating, printing, casting, Langmuir-Blodgett (LB) deposition, or the like. When the HTL is formed by vacuum deposition or spin coating, the conditions for deposition and coating may be similar to those for the formation of the HIL. However, the conditions for the vacuum or solution deposition may vary, according to the compound that is used to form the HTL.

The HTL may be formed of any compound that is commonly used to form a HTL. Compounds that can be suitably used are disclosed for example in Yasuhiko Shirota and Hiroshi Kageyama, Chem. Rev. 2007, 107, 953-1010.

Examples of the compound that may be used to form the HTL are: carbazole derivatives, such as N-phenylcarbazole or polyvinylcarbazole; benzidine derivatives, such as N,N'-bis(3-methylphenyl)-N,N'-diphenyl-[1,1-biphenyl]-4,4'-diamine (TPD), or N,N'-di(naphthalen-1-yl)-N,N'-diphenyl benzidine (alpha-NPD); and triphenylamine-based compound, such as 4,4',4"-tris(N-carbazolyl)triphenylamine (TCTA). Among these compounds, TCTA can transport holes and inhibit excitons from being diffused into the EML.

According to one embodiment of the present invention, the hole transport layer may comprise a substantially covalent matrix compound as described above.

According to a preferred embodiment of the present invention, the hole injection layer and the hole transport layer may comprise an identical substantially covalent matrix compound as described above.

According to one embodiment of the present invention, the hole transport layer may comprise a compound of formula (VI) or (VII) as described above.

According to a preferred embodiment of the present invention, the hole injection layer and the hole transport layer may comprise an identical compound of formula (VI) or (VII) as described above.

According to a preferred embodiment of the present invention, the p-type charge generation layer, the hole injection layer and the hole transport layer may comprise an identical substantially covalent matrix compound.

According to a preferred embodiment of the present invention, the p-type charge generation layer, the hole injection layer and the hole transport layer may comprise an identical an identical compound of formula (VI) or (VII) as described above.

According to a preferred embodiment of the present invention, the hole injection layer, the hole transport layer and the p-type charge generation layer may comprise an identical compound of formula (VI) or (VII) as described above; and the hole injection layer and the p-type charge generation layer may comprise an identical compound of formula (I) or (IV), as described above.

The thickness of the HTL may be in the range of about 5 nm to about 250 nm, preferably, about 10 nm to about 200 nm, further about 20 nm to about 190 nm, further about 40 nm to about 180 nm, further about 60 nm to about 170 nm, further about 80 nm to about 160 nm, further about 100 nm to about 160 nm, further about 120 nm to about 140 nm. A preferred thickness of the HTL may be 170 nm to 200 nm.

When the thickness of the HTL is within this range, the HTL may have excellent hole transporting characteristics, without a substantial penalty in driving voltage.

### Electron blocking layer

The function of an electron blocking layer (EBL) is to prevent electrons from being transferred from an emission layer to the hole transport layer and thereby confine electrons to the emission layer. Thereby, efficiency, operating voltage and/or lifetime are improved. Typically, the electron blocking layer comprises a triarylamine compound. The triarylamine compound may have a LUMO level closer to vacuum level than the LUMO level of the hole transport layer. The electron blocking layer may have a HOMO level that is further away from vacuum level compared to the HOMO level of the hole transport layer. The thickness of the electron blocking layer may be selected between 2 and 20 nm.

If the electron blocking layer has a high triplet level, it may also be described as triplet control layer.

The function of the triplet control layer is to reduce quenching of triplets if a phosphorescent green or blue emission layer is used. Thereby, higher efficiency of light emission from a phosphorescent emission layer can be achieved. The triplet control layer is selected from triarylamine compounds with a triplet level above the triplet level of the phosphorescent emitter in the adjacent emission layer. Suitable compounds for the triplet control layer, in particular the triarylamine compounds, are described in EP 2 722 908 A1.

### Photoactive layer (PAL)

According to an embodiment of the present invention, the organic electronic device may further comprise a photoactive layer, wherein the photoactive layer is arranged between the anode layer and the cathode layer.

The photoactive layer converts an electrical current into photons or photons into an electrical current.

The PAL may be formed on the HTL by vacuum deposition, spin coating, slot-die coating, printing, casting, LB deposition, or the like. When the PAL is formed using vacuum deposition or spin coating, the conditions for deposition and coating may be similar to those for the formation of the HIL. However, the conditions for deposition and coating may vary, according to the compound that is used to form the PAL.

According to one embodiment of the present invention, the photoactive layer does not comprise the compound of formula (I).

The photoactive layer may be a light-emitting layer or a light-absorbing layer.

### Emission layer (EML)

According to an embodiment of the present invention, the organic electronic device may further comprise an emission layer, wherein the emission layer is arranged between the anode layer and the cathode layer.

The EML may be formed on the HTL by vacuum deposition, spin coating, slot-die coating, printing, casting, LB deposition, or the like. When the EML is formed using vacuum deposition or spin coating, the conditions for deposition and coating may be similar to those for the formation of the HIL. However, the conditions for deposition and coating may vary, according to the compound that is used to form the EML.

According to one embodiment of the present invention, the emission layer does not comprise the compound of formula (I).

The emission layer (EML) may be formed of a combination of a host and an emitter dopant. Example of the host are Alq3, 4,4'-N,N'-dicarbazole-biphenyl (CBP), poly(n-vinylcarbazole) (PVK), 9,10-di(naphthalene-2-yl)anthracene (ADN), 4,4',4''-tris(carbazol-9-yl)-triphenylamine(TCTA), 1,3,5-tris(N-phenylbenzimidazole-2-yl)benzene (TPBI), 3-tert-butyl-9,10-di-2-naphthylanthracenee (TBADN), distyrylarylene (DSA) and bis(2-(2-hydroxyphenyl)benzo-thiazolate)zinc (Zn(BTZ)2).

The emitter dopant may be a phosphorescent or fluorescent emitter. Phosphorescent emitters and emitters which emit light via a thermally activated delayed fluorescence (TADF) mechanism may be preferred due to their higher efficiency. The emitter may be a small molecule or a polymer.

Examples of red emitter dopants are PtOEP, Ir(piq)3, and Btp2lr(acac), but are not limited thereto. These compounds are phosphorescent emitters, however, fluorescent red emitter dopants could also be used.

Examples of phosphorescent green emitter dopants are Ir(ppy)3 (ppy = phenylpyridine), Ir(ppy)2(acac), Ir(mpyp)3.

Examples of phosphorescent blue emitter dopants are F2Irpic, (F2ppy)2Ir(tmd) and Ir(dfppz)3 and ter-fluorene. 4.4'-bis(4-diphenyl amiostyryl)biphenyl (DPAVBi), 2,5,8,11-tetra-tert-butyl perylene (TBPe) are examples of fluorescent blue emitter dopants.

The amount of the emitter dopant may be in the range from about 0.01 to about 50 parts by weight, based on 100 parts by weight of the host. Alternatively, the emission layer may consist of a light-emitting polymer. The EML may have a thickness of about 10 nm to about 100 nm, for example, from about 20 nm to about 60 nm. When the thickness of the EML is within this range, the EML may have excellent light emission, without a substantial penalty in driving voltage.

### Hole blocking layer (HBL)

A hole blocking layer (HBL) may be formed on the EML, by using vacuum deposition, spin coating, slot-die coating, printing, casting, LB deposition, or the like, in order to prevent the diffusion of holes into the ETL. When the EML comprises a phosphorescent dopant, the HBL may have also a triplet exciton blocking function.

The HBL may also be named auxiliary ETL or a-ETL.

When the HBL is formed using vacuum deposition or spin coating, the conditions for deposition and coating may be similar to those for the formation of the HIL. However, the conditions for deposition and coating may vary, according to the compound that is used to form the HBL. Any compound that is commonly used to form a HBL may be used. Examples of compounds for forming the HBL include oxadiazole derivatives, triazole derivatives, phenanthroline derivatives and azine derivatives, preferably triazine or pyrimidine derivatives.

The HBL may have a thickness in the range from about 5 nm to about 100 nm, for example, from about 10 nm to about 30 nm. When the thickness of the HBL is within this range, the HBL may have excellent hole-blocking properties, without a substantial penalty in driving voltage.

### Electron transport layer (ETL)

The organic electronic device according to the present invention may further comprise an electron transport layer (ETL).

According to another embodiment of the present invention, the electron transport layer may further comprise an azine compound, preferably a triazine compound or a pyrimidine compound.

In one embodiment, the electron transport layer may further comprise a dopant selected from an alkali organic complex, preferably LiQ.

The thickness of the ETL may be in the range from about 15 nm to about 50 nm, for example, in the range from about 20 nm to about 40 nm. When the thickness of the EIL is within this range, the ETL may have satisfactory electron-injecting properties, without a substantial penalty in driving voltage.

According to another embodiment of the present invention, the organic electronic device may further comprise a hole blocking layer and an electron transport layer, wherein the hole blocking layer and the electron transport layer comprise an azine compound. Preferably, the azine compound is a triazine compound.

### Electron injection layer (EIL)

An optional EIL, which may facilitates injection of electrons from the cathode, may be formed on the ETL, preferably directly on the electron transport layer. Examples of materials for forming the EIL include lithium 8-hydroxyquinolinolate (LiQ), LiF, NaCl, CsF, Li2O, BaO, Ca, Ba, Yb, Mg which are known in the art. Deposition and coating conditions for forming the EIL are similar to those for formation of the HIL, although the deposition and coating conditions may vary, according to the material that is used to form the EIL.

The thickness of the EIL may be in the range from about 0.1 nm to about 10 nm, for example, in the range from about 0.5 nm to about 9 nm. When the thickness of the EIL is within this range, the EIL may have satisfactory electron-injecting properties, without a substantial penalty in driving voltage.

### Cathode layer

The cathode layer is formed on the ETL or optional EIL. The cathode layer may be formed of a metal, an alloy, an electrically conductive compound, or a mixture thereof. The cathode electrode may have a low work function. For example, the cathode layer may be formed of lithium (Li), magnesium (Mg), aluminum (Al), aluminum (Al)-lithium (Li), calcium (Ca), barium (Ba), ytterbium (Yb), magnesium (Mg)-indium (In), magnesium (Mg)-silver (Ag), or the like. Alternatively, the cathode electrode may be formed of a transparent conductive oxide, such as ITO or IZO.

The thickness of the cathode layer may be in the range from about 5 nm to about 1000 nm, for example, in the range from about 10 nm to about 100 nm. When the thickness of the cathode layer is in the range from about 5 nm to about 50 nm, the cathode layer may be transparent or semitransparent even if formed from a metal or metal alloy.

In a preferred embodiment, the cathode layer comprises a metal or metal alloy and is transparent.

It is to be understood that the cathode layer is not part of an electron injection layer or the electron transport layer.

### Organic light-emitting diode (OLED)

The organic electronic device according to the invention may be an organic light-emitting diode.

According to one aspect of the present invention, there is provided an organic light-emitting diode (OLED) comprising: a substrate; an anode layer formed on the substrate; a charge generation layer according to invention, at least one emission layer and a cathode layer.

According to one aspect of the present invention, there is provided an organic light-emitting diode (OLED) comprising: a substrate; an anode layer formed on the substrate; a charge generation layer according to invention, at least a first and a second emission layers and a cathode layer, wherein the charge generation layer is arranged between the first and the second emission layer.

According to one aspect of the present invention, there is provided an organic light-emitting diode (OLED) comprising: a substrate; an anode layer formed on the substrate; a hole injection layer which may comprise a compound of formula (I), a hole transport layer, an emission layer, an electron transport layer, a n-type charge generation layer, a p-type charge generation layer comprising a compound of formula (I), a hole transport layer, an optional electron injection layer and a cathode layer.

According to various embodiments of the present invention, there may be provided OLEDs layers arranged between the above mentioned layers, on the substrate or on the top electrode.

According to one aspect, the OLED may comprise a layer structure of a substrate that is adjacent arranged to an anode electrode, the anode electrode is adjacent arranged to a first hole injection layer, the first hole injection layer is adjacent arranged to a first hole transport layer, the first hole transport layer is adjacent arranged to a first electron blocking layer, the first electron blocking layer is adjacent arranged to a first emission layer, the first emission layer is adjacent arranged to a first electron transport layer, the first electron transport layer is adjacent arranged to an n-type charge generation layer, the n-type charge generation layer is adjacent arranged to a p-type charge generating layer, the p-type charge generating layer is adjacent arranged to a second hole transport layer, the second hole transport layer is adjacent arranged to a second electron blocking layer, the second electron blocking layer is adjacent arranged to a second emission layer, between the second emission layer and the cathode electrode an optional electron transport layer and/or an optional electron injection layer are arranged.

### Organic electronic device

The organic electronic device according to the invention may be a light emitting device.

According to another aspect of the present invention, there is provided a method of manufacturing an organic electronic device, the method using:
- at least one deposition source, preferably two deposition sources and more preferred at least three deposition sources.

The methods for deposition that can be suitable comprise:
- deposition via vacuum thermal evaporation;
- deposition via solution processing, preferably the processing is selected from spin-coating, printing, casting; and/or
- slot-die coating.

According to various embodiments of the present invention, there is provided a method using:
- a first deposition source to release the compound of formula (I) according to the invention, and
- a second deposition source to release the substantially covalent matrix compound;
- a third deposition source to release the n-CGL matrix compound;
- a fourth deposition source to release the n-CGL dopant;

the method comprising the steps of forming the p-type charge generation layer; whereby for an organic light-emitting diode (OLED):
   - the p-type charge generation layer is formed by releasing the compound of formula (I) according to the invention from the first deposition source and the substantially covalent matrix compound from the second deposition source;
the method comprising the steps of forming the n-type charge generation layer; whereby for an organic light-emitting diode (OLED):
   - the n-type charge generation layer is formed by releasing the n-CGL matrix compound according to the invention from the third deposition source and n-CGL dopant from the fourth deposition source.

According to various embodiments of the present invention, the method may further include forming on the anode electrode, at least one layer selected from the group consisting of forming a hole transport layer or forming a hole blocking layer, an emission layer and a n-type charge generation layer between the anode electrode and the cathode layer.

According to another aspect of the invention, it is provided an electronic device comprising at least one organic light emitting device according to any embodiment described throughout this application, preferably, the electronic device comprises the organic light emitting diode in one of embodiments described throughout this application. More preferably, the electronic device is a display device.

Hereinafter, the embodiments are illustrated in more detail with reference to examples. However, the present disclosure is not limited to the following examples. Reference will now be made in detail to the exemplary aspects.

### Description of the Drawings

The aforementioned components, as well as the claimed components and the components to be used in accordance with the invention in the described embodiments, are not subject to any special exceptions with respect to their size, shape, material selection and technical concept such that the selection criteria known in the pertinent field can be applied without limitations.

Additional details, characteristics and advantages of the object of the invention are disclosed in the dependent claims and the following description of the respective figures which in an exemplary fashion show preferred embodiments according to the invention. Any embodiment does not necessarily represent the full scope of the invention, however, and reference is made therefore to the claims and herein for interpreting the scope of the invention. It is to be understood that both the foregoing general description and the following detailed description are exemplary and explanatory only and are intended to provide further explanation of the present invention as claimed.
FIG. 1 is a schematic sectional view of an OLED comprising a charge generation layer, according to an exemplary embodiment of the present invention.
FIG. 2 is a schematic sectional view of a stacked OLED comprising a charge generation layer, according to an exemplary embodiment of the present invention.

Hereinafter, the figures are illustrated in more detail with reference to examples. However, the present disclosure is not limited to the following figures.

Herein, when a first element is referred to as being formed or disposed "on" or "onto" a second element, the first element can be disposed directly on the second element, or one or more other elements may be disposed there between. When a first element is referred to as being formed or disposed "directly on" or "directly onto" a second element, no other elements are disposed there between.

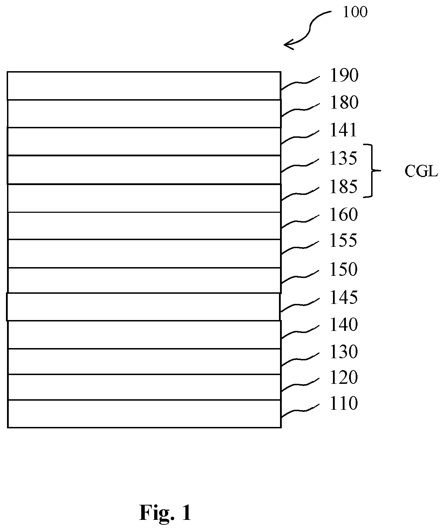

Fig. 1 is a schematic sectional view of an OLED 100, according to one exemplary embodiment of the present invention.

Referring to Fig. 1 the OLED 100 includes a substrate 110, an anode layer 120, a hole injection layer (HIL) 130, a first hole transport layer (HTL1) 140, an electron blocking layer (EBL) 145, an emission layer (EML) 150, a hole blocking layer (HBL) 155, an electron transport layer (ETL) 160, an n-type charge generation layer (n-CGL) 185, a p-type charge generation layer (p-GCL) 135 which comprises a compound of Formula (I), a second hole transport layer (HTL2) 141, and electron injection layer (EIL) 180 and a cathode layer 190. The HIL may comprise a compound of Formula (I).

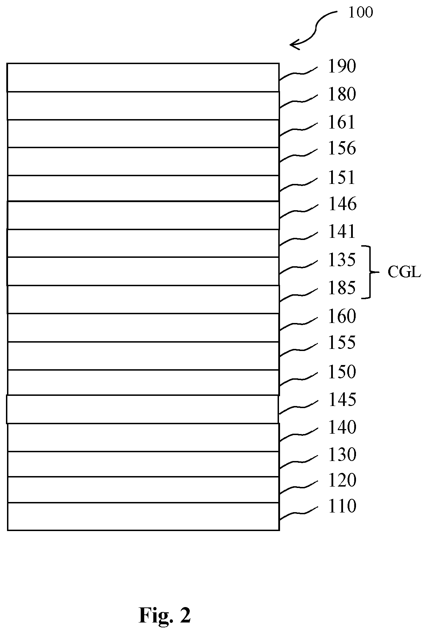

Fig. 2 is a schematic sectional view of a stacked OLED 100, according to another exemplary embodiment of the present invention. Fig. 2 differs from Fig. 1 in that the OLED 100 of Fig. 1 further comprises a second emission layer.

Referring to Fig. 2 the OLED 100 includes a substrate 110, an anode layer 120, a hole injection layer (HIL) 130, a first hole transport layer (HTL) 140, a first electron blocking layer (EBL) 145, a first emission layer (EML) 150, an optional first hole blocking layer (HBL) 155, a first electron transport layer (ETL) 160, an n-type charge generation layer (n-CGL) 185, a p-type charge generation layer (p-GCL) 135 which comprises compound of Formula (I), a second hole transport layer (HTL) 141, a second electron blocking layer (EBL) 146, a second emission layer (EML) 151, an optional second hole blocking layer (HBL) 156, a second electron transport layer (ETL) 161, an electron injection layer (EIL) 181 and a cathode layer 190. The HIL may comprise a compound of Formula (I).

In the description above the method of manufacture an OLED 100 of the present invention is started with a substrate 110 onto which an anode layer 120 is formed, on the anode layer 120, a hole injection layer 130, a first hole transport layer 140, optional a first electron blocking layer 145, a first emission layer 150, optional a first hole blocking layer 155, optional at least one first electron transport layer 160, an n-CGL 185, a p-CGL 135, a second hole transport layer 141, optional a second electron blocking layer 146, a second emission layer 151, an optional second hole blocking layer 156, an optional at least one second electron transport layer 161, an optional electron injection layer 180 and a cathode layer 190 are formed, in that order or the other way around.

While not shown in Fig. 1 and 2, a capping and/or a sealing layer may further be formed on the cathode layer 190, in order to seal the organic electronic device 100. In addition, various other modifications may be applied thereto.

Hereinafter, one or more exemplary embodiments of the present invention will be described in detail with, reference to the following examples. However, these examples are not intended to limit the purpose and scope of the one or more exemplary embodiments of the present invention.

### Detailed description

The invention is furthermore illustrated by the following examples which are illustrative only and non-binding.

Compounds of formula (I) may be prepared as described in EP2180029A1 and WO2016097017A1.

### HOMO and LUMO

The HOMO and LUMO are calculated with the program package TURBOMOLE V6.5 (TURBOMOLE GmbH, Litzenhardtstrasse 19, 76135 Karlsruhe, Germany). The optimized geometries and the HOMO and LUMO energy levels of the molecular structures are determined by applying the hybrid functional B3LYP with a 6-31G* basis set in the gas phase. If more than one conformation is viable, the conformation with the lowest total energy is selected.

### Thermogravimetric analysis

The term "TGA5%" denotes the temperature at which 5 % weight loss occurs during thermogravimetric analysis and is measured in °C.

The TGA5% value may be determined by by heating a 9-11 mg sample in a thermogravimetric analyser at a heating rate of 10 K/min in an open 100 µL aluminium pan, under a stream of nitrogen at a flow rate of 20 mL/min in the balance area and of 30 mL/min in the oven area.

The TGA5% value may provide an indirect measure of the volatility and/or decomposition temperature of a compound. In first approximation, the higher the TGA5% value the lower is the volatility of a compound and/or the higher the decomposition temperature.

According to one embodiment, the TGA5% value of compound of formula (I) is selected in the range of ≥ 270 °C and ≤ 450 °C; preferably of ≥ 280 °C and ≤ 440 °C, also preferred of ≥ 295 °C and ≤ 430 °C.

### Glass transition temperature

The glass transition temperature, also named Tg, is measured in °C and determined by Differential Scanning Calorimetry (DSC).

The glass transition temperature is measured under nitrogen and using a heating rate of 10 K per min in a Mettler Toledo DSC 822e differential scanning calorimeter as described in DIN EN ISO 11357, published in March 2010.

### General procedure for fabrication of OLEDs with a transparent anode layer (bottom emission device

For OLEDs, see Table 5, a 15Ω /cm² glass substrate with 90 nm ITO (available from Corning Co.) was cut to a size of 50 mm x 50 mm x 0.7 mm, ultrasonically washed with isopropyl alcohol for 5 minutes and then with pure water for 5 minutes, and washed again with UV ozone for 30 minutes, to prepare the anode.

Then a substantially covalent matrix compound and a compound of formula (1) or a comparative compound was vacuum co-deposited on the anode, to form a HIL having a thickness of 10 nm. The composition of the HIL can be seen in Table 5.

Then the same substantially covalent matrix compound was vacuum deposited on the HIL, to form a first HTL having a thickness of 128 nm.

Then N,N-di([1,1'-biphenyl]-4-yl)-3'-(9H-carbazol-9-yl)-[1,1'-biphenyl]-4-amine was vacuum deposited on the first HTL, to form a first electron blocking layer (EBL1) having a thickness of 5 nm.

Then 97 vol.-% H09 (Sun Fine Chemicals, Korea) as EML host and 3 vol.-% BD200 (Sun Fine Chemicals, Korea) as fluorescent blue dopant were deposited on the EBL, to form a first emission layer (EML1) with a thickness of 20 nm.

Then a first electron transport layer (ETL1) is formed on the first emission layer by depositing 2,4-diphenyl-6-(3'-(triphenylen-2-yl)-[1,1'-biphenyl]-3-yl)-1,3,5-triazine having a thickness of 25 nm.

Then, the n-CGL having a thickness of 10 nm is formed on the ETL by co-depositing 99 vol.-% 2,2'-(1,3-Phenylene)bis[9-phenyl-1,10-phenanthroline] and 3 vol.-% Li.

Then, the p-CGL having a thickness of 10 nm is formed on the n-CGL by co-depositing a substantially covalent matrix compound and a compound of formula (I). The composition of the p-CGL can be seen in Table 5.

Then, a second hole transport layer (HTL2) having a thickness of 25 nm is formed on the p-CGL by depositing a substantially covalent matrix compound as in the first hole transport layer. The composition of the second hole transport layer is the same as of the first hole transport layer.

Then N,N-di([1,1'-biphenyl]-4-yl)-3'-(9H-carbazol-9-yl)-[1,1'-biphenyl]-4-amine was vacuum deposited on the second HTL, to form a second electron blocking layer (EBL2) having a thickness of 5 nm.

Then 97 vol.-% H09 (Sun Fine Chemicals, Korea) as EML host and 3 vol.-% BD200 (Sun Fine Chemicals, Korea) as fluorescent blue dopant were deposited on the EBL, to form a second emission layer (EML2) with a thickness of 20 nm.

Then a second electron transport layer (ETL2) is formed on the second emission layer by depositing 2,4-diphenyl-6-(3'-(triphenylen-2-yl)-[1,1'-biphenyl]-3-yl)-1,3,5-triazine having a thickness of 26 nm.

Then, the electron injection layer having a thickness of 10 nm is formed on the second electron transport layer by co-depositing 99 vol.-% 2,2'-(1,3-Phenylene)bis[9-phenyl-1,10-phenanthroline] and 3 vol.-% Li.

Then Al is vacuum deposited on the electron injection layer at a rate of 0.01 to 1 Å/s at 10⁻⁷ mbar to form a cathode layer with a thickness of 100 nm.

The OLED stack is protected from ambient conditions by encapsulation of the device with a glass slide. Thereby, a cavity is formed, which includes a getter material for further protection.

### General procedure for fabrication of OLEDs with a transparent cathode layer (top emission device)

For OLEDs comprising a CGL, see Table 6, a glass substrate was cut to a size of 50 mm x 50 mm x 0.7 mm, ultrasonically washed with isopropyl alcohol for 5 minutes and then with pure water for 5 minutes, and washed again with UV ozone for 30 minutes, to prepare the substrate.

Then, the anode layer having a thickness of 100 nm is formed on the substrate by vacuum depositing Ag at a rate of 0.01 to 1 Å/s at 10⁻⁷ mbar.

Then, a hole injection layer (HIL) having a thickness of 10 nm is formed on the anode layer by co-depositing a substantially covalent matrix compound and a compound of formula (I) or a comparative compound. The composition of the HIL can be seen in Table 6.

Then, a first hole transport layer (HTL1) having a thickness of 34 nm is formed on the HIL by depositing the substantially covalent matrix compound. The substantially covalent matrix compound is the same as in the HIL.

Then, an electron blocking layer (EBL) having a thickness of 5 nm is formed on the HTL1 by depositing N-([1,1'-biphenyl]-4-yl)-9,9-diphenyl-N-(4-(triphenylsilyl)phenyl)-9H-fluoren-2-amine.

Then, a first emission layer (EML1) having a thickness of 20 nm is formed on the EBL by co-depositing 97 vol.-% H09 (Sun Fine Chemicals, Korea) as EML host and 3 vol.-% BD200 (Sun Fine Chemicals, Korea) as fluorescent blue dopant.

Then, a hole blocking layer (HBL) is formed with a thickness of 5 nm is formed on the first emission layer by depositing 2-(3'-(9,9-dimethyl-9H-fluoren-2-yl)-[1,1'-biphenyl]-3-yl)-4,6-diphenyl-1,3,5-triazine.

Then, an electron transporting layer (ETL) having a thickness of 20 nm is formed on the hole blocking layer by co-depositing 50 wt.-% 2-([1,1'-biphenyl]-4-yl)-4-(9,9-diphenyl-9H-fluoren-4-yl)-6-phenyl-1,3,5-triazine and 50 wt.-% LiQ.

Then, the n-CGL having a thickness of 10 nm is formed on the ETL by co-depositing 99 vol.-% 2,2'-(1,3-Phenylene)bis[9-phenyl-1,10-phenanthroline] and 1 vol.-% Li.

Then, the p-CGL is formed on the n-CGL by co-depositing a substantially covalent matrix compound and a compound of formula (I) having a thickness of 10 nm. The composition of the p-CGL can be seen in Table 6.

Then, a second hole transport layer (HTL2) having a thickness of 81 nm is formed on the p-CGL by depositing a substantially covalent matrix compound. The composition of the second hole transport layer is the same as of the first hole transport layer.

Then, an electron injection layer (EIL) having a thickness of 2 nm is formed on the HTL2 by depositing Yb.

Then, the cathode layer having a thickness of 13 nm is formed on the EIL by co-depositing Ag:Mg (90:10 vol.-%) at a rate of 0.01 to 1 Å/s at 10⁻⁷ mbar.

Then, a capping layer having a thickness of 75 nm is formed on the cathode layer by depositing compound of formula F3.

The OLED stack is protected from ambient conditions by encapsulation of the device with a glass slide. Thereby, a cavity is formed, which includes a getter material for further protection.

To assess the performance of the inventive examples compared to the prior art, the current efficiency is measured at 20°C. The current-voltage characteristic is determined using a Keithley 2635 source measure unit, by sourcing a voltage in V and measuring the current in mA flowing through the device under test. The voltage applied to the device is varied in steps of 0.1V in the range between 0V and 10V. Likewise, the luminance-voltage characteristics and CIE coordinates are determined by measuring the luminance in cd/m² using an Instrument Systems CAS-140CT array spectrometer (calibrated by Deutsche Akkreditierungsstelle (DAkkS)) for each of the voltage values. The cd/A efficiency at 10 mA/cm2 is determined by interpolating the luminance-voltage and current-voltage characteristics, respectively.

In bottom emission devices, the emission is predominately Lambertian and quantified in percent external quantum efficiency (EQE). To determine the efficiency EQE in % the light output of the device is measured using a calibrated photodiode at 10 mA/cm2.

In top emission devices, the emission is forward directed, non-Lambertian and also highly dependent on the micro-cavity. Therefore, the efficiency EQE will be higher compared to bottom emission devices. To determine the efficiency EQE in % the light output of the device is measured using a calibrated photodiode at 10 mA/cm².

Lifetime LT of the device is measured at ambient conditions (20°C) and 30 mA/cm², using a Keithley 2400 sourcemeter, and recorded in hours.

The brightness of the device is measured using a calibrated photo diode. The lifetime LT is defined as the time till the brightness of the device is reduced to 97 % of its initial value.

The increase in operating voltage U over time "U rise (100-1h)" is measured by determining the difference in operating voltage at 30mA/cm² after 1 hour and after 50 hours.

The increase in operating voltage U over time "U rise (400-1h)" is measured by determining the difference in operating voltage at 30mA/cm² after 1 hour and after 400 hours.

### Technical Effect of the invention

In Table 2 are shown LUMO levels for Examples A1 to A82. LUMO levels were calculated with the program package TURBOMOLE V6.5 (TURBOMOLE GmbH, Litzenhardtstrasse 19, 76135 Karlsruhe, Germany) by applying the hybrid functional B3LYP with a 6-31G* basis set in the gas phase

**Table 2: Structure of the inventive compounds A1 to A82 and their LUMOs**

| | A¹ | A² | A³ | LUMO [eV] |
|---|---|---|---|---|
| A1 | 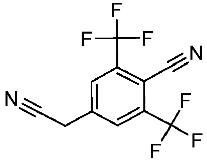 | 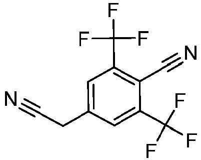 | 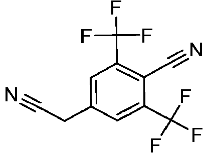 | -5.36 |
| A2 | 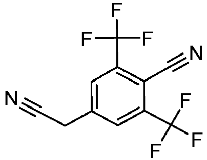 | 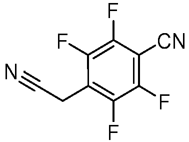 | 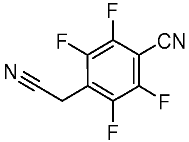 | -5.25 |
| A3 | 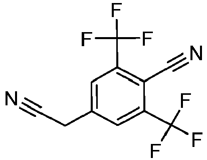 | 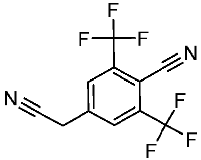 | 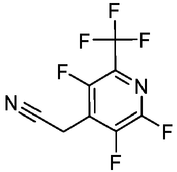 | -5.34 |
| A4 | 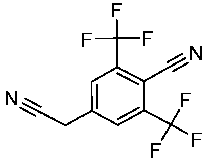 | 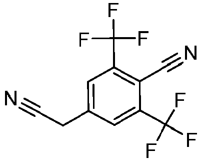 | 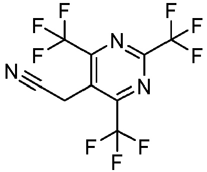 | -5.29 |
| A5 | 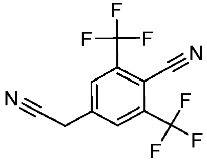 | 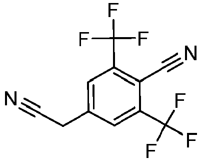 | 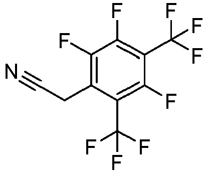 | -5.19 |
| A6 | 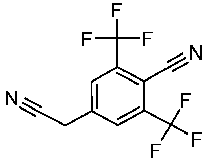 | 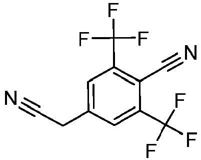 | 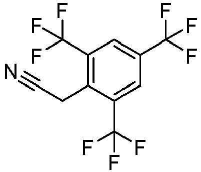 | -5.05 |
| A7 | 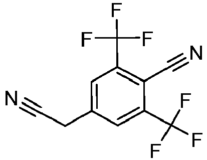 | 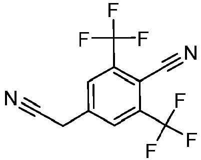 | 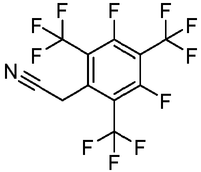 | -5.15 |
| A8 | 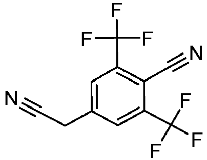 | 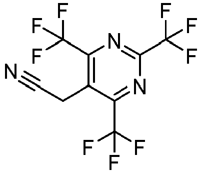 | 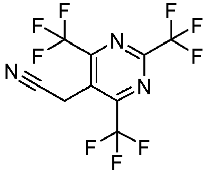 | -5.22 |
| A9 | 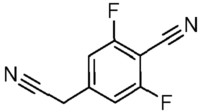 | 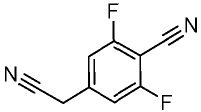 | 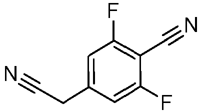 | -5.14 |
| A10 | 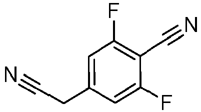 | 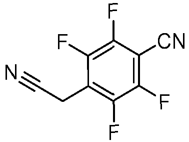 | 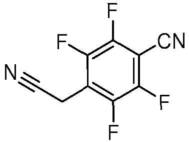 | -5.17 |
| A11 | 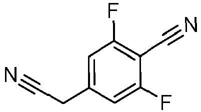 | 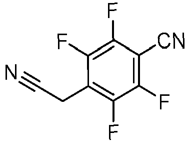 | 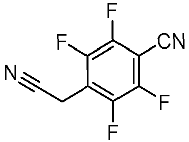 | -5.17 |
| A12 | 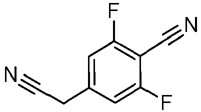 | 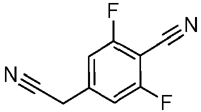 | 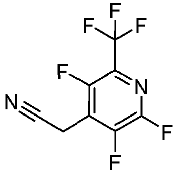 | -5.19 |
| A13 | 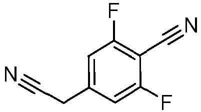 | 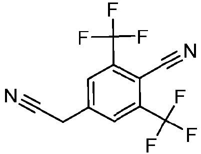 | 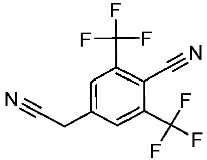 | -5.29 |
| A14 | 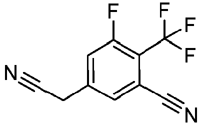 | 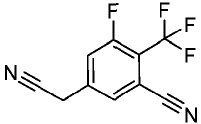 | 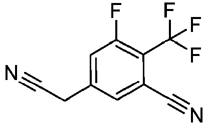 | -5.20 |
| A15 | 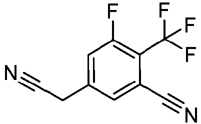 | 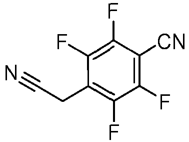 | 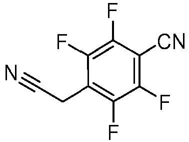 | -5.19 |
| A16 | 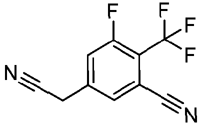 | 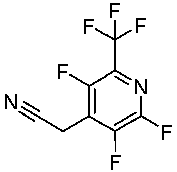 | 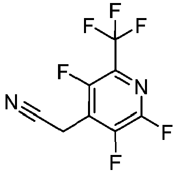 | -5.26 |
| A17 | 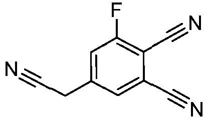 | 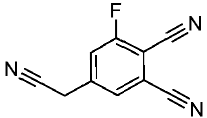 | 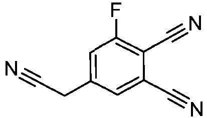 | -5.51 |
| A18 | 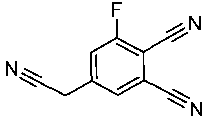 | 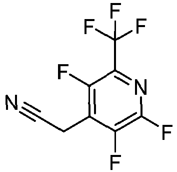 | 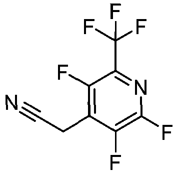 | -5.36 |
| A19 | 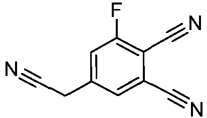 | 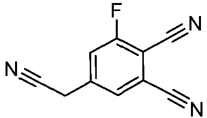 | 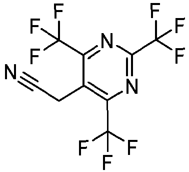 | -5.39 |
| A20 | 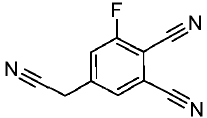 | 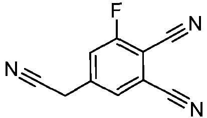 | 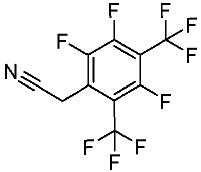 | -5.29 |
| A21 | 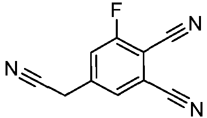 | 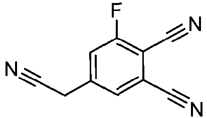 | 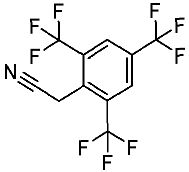 | -5.15 |
| A22 | 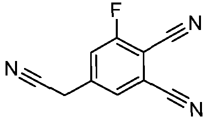 | 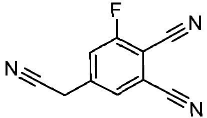 | 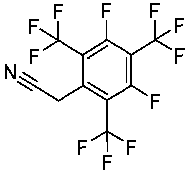 | -5.25 |
| A23 | 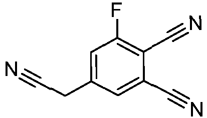 | 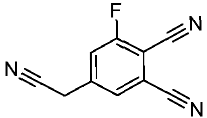 | 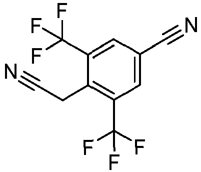 | -5.24 |
| A24 | 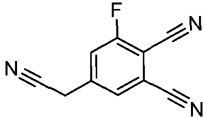 | 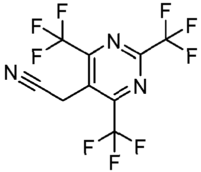 | 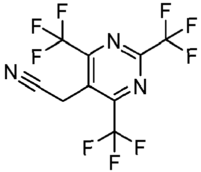 | -5.27 |
| A25 | 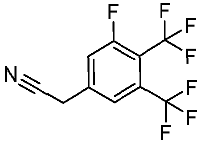 | 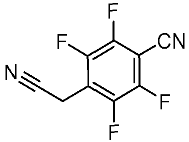 | 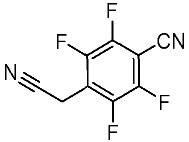 | -5.12 |
| A26 | 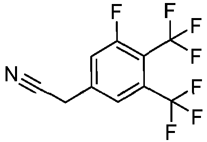 | 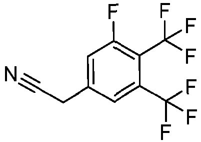 | 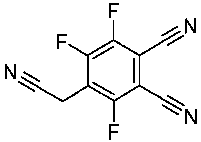 | -5.16 |
| A27 | 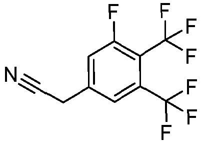 | 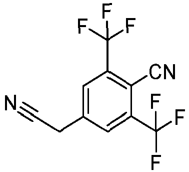 | 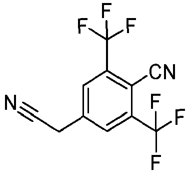 | -5.23 |
| A28 | 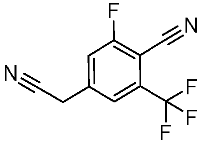 | 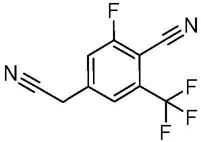 | 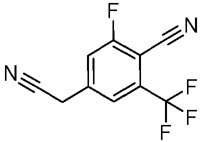 | -5.26 |
| A29 | 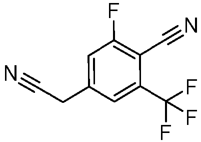 | 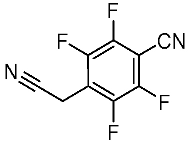 | 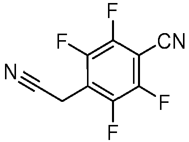 | -5.12 |
| A30 | 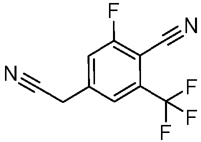 | 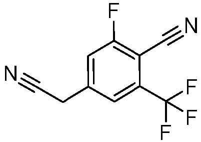 | 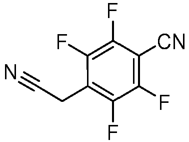 | -5.24 |
| A31 | 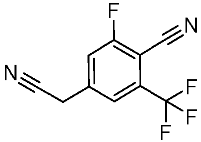 | 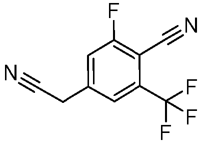 | 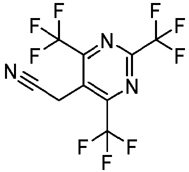 | -5.22 |
| A32 | 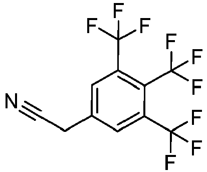 | 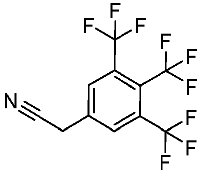 | 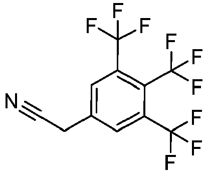 | -5.06 |
| A33 | 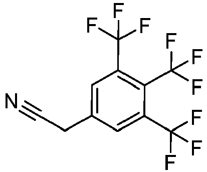 | 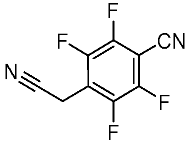 | 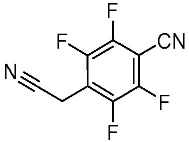 | -5.15 |
| A34 | 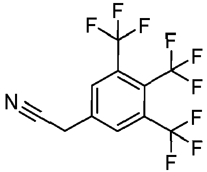 | 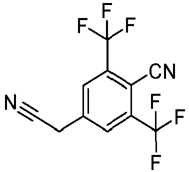 | 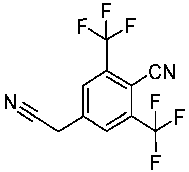 | -5.26 |
| A35 | 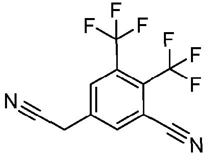 | 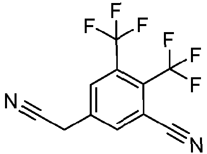 | 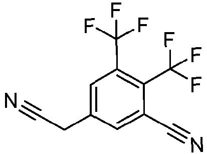 | -5.33 |
| A36 | 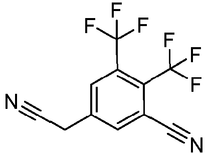 | 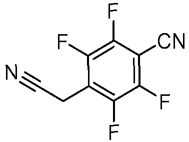 | 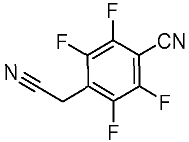 | -5.24 |
| A37 | 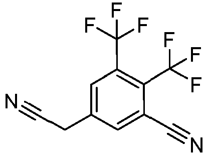 | 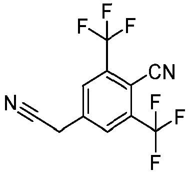 | 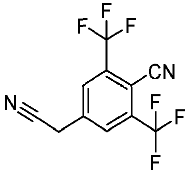 | -5.35 |
| A38 | 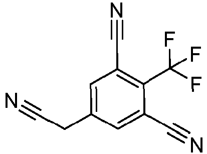 | 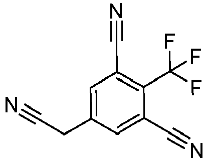 | 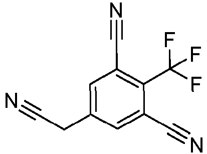 | -5.55 |
| A39 | 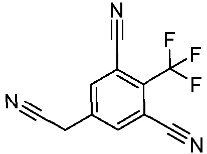 | 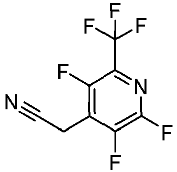 | 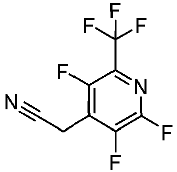 | -5.38 |
| A40 | 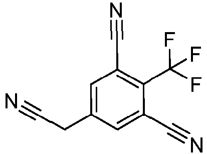 | 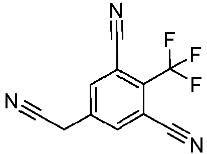 | 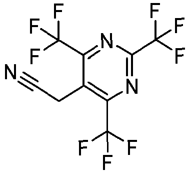 | -5.42 |
| A41 | 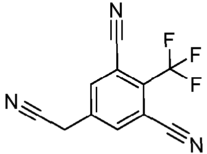 | 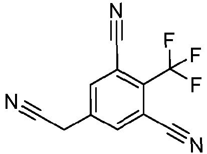 | 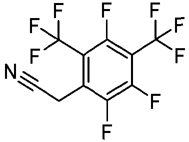 | -5.32 |
| A42 | 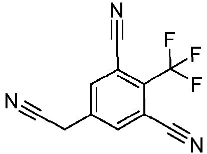 | 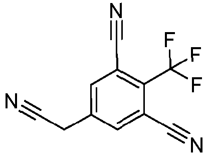 | 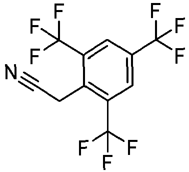 | -5.18 |
| A43 | 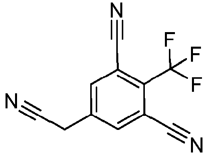 | 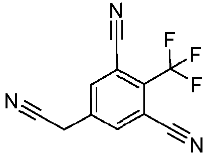 | 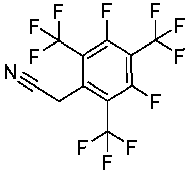 | -5.28 |
| A44 | 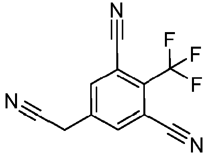 | 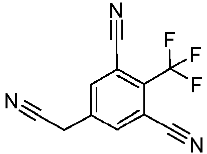 | 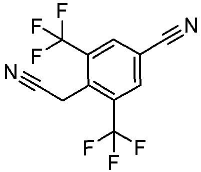 | -5.26 |
| A45 | 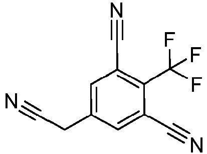 | 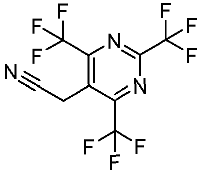 | 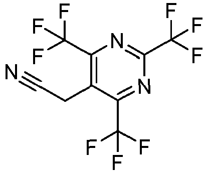 | -5.27 |
| A46 | 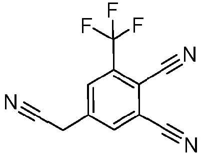 | 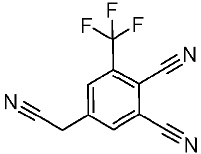 | 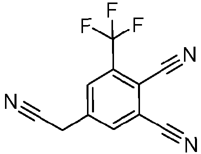 | -5.60 |
| A47 | 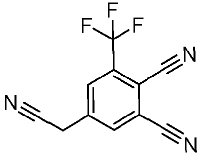 | 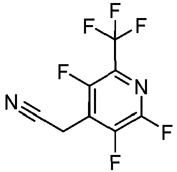 | 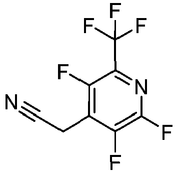 | -5.39 |
| A48 | 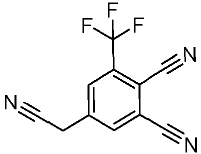 | 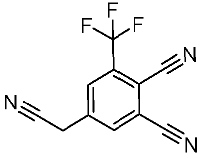 | 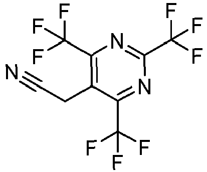 | -5.45 |
| A49 | 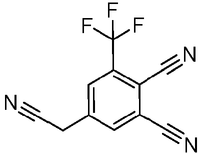 | 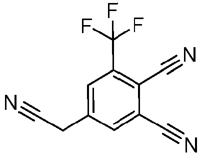 | 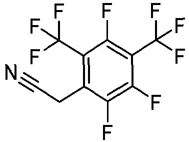 | -5.35 |
| A50 | 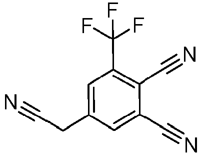 | 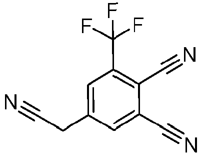 | 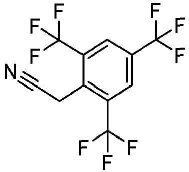 | -5.21 |
| A51 | 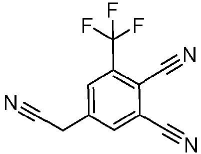 | 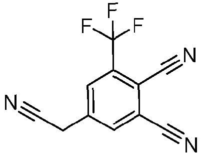 | 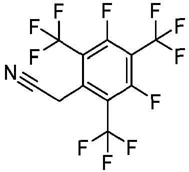 | -5.31 |
| A52 | 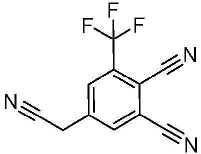 | 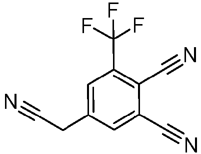 | 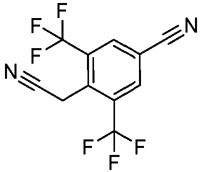 | -5.30 |
| A53 | 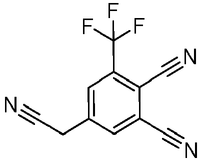 | 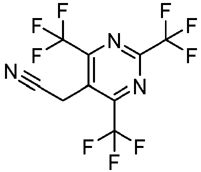 | 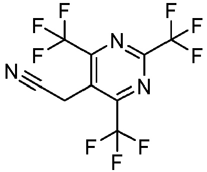 | -5.30 |
| A54 | 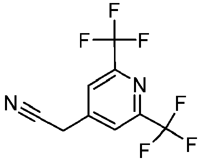 | 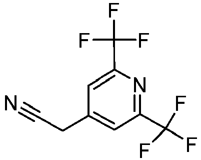 | 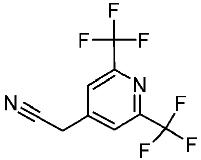 | -5.33 |
| A55 | 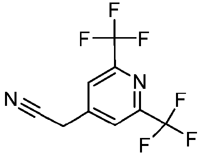 | 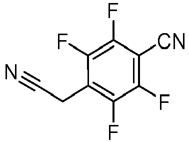 | 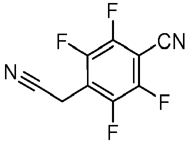 | -5.24 |
| A56 | 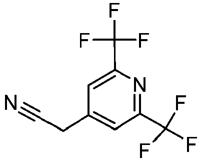 | 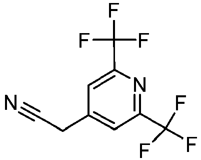 | 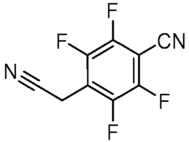 | -5.28 |
| A57 | 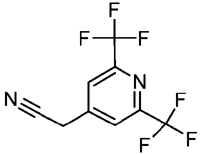 | 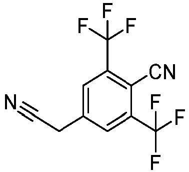 | 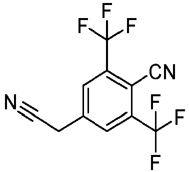 | -5.35 |
| A58 | 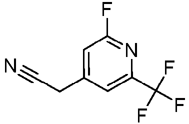 | 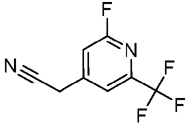 | 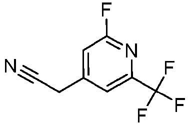 | -5.19 |
| A59 | 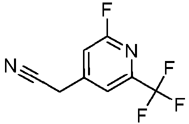 | 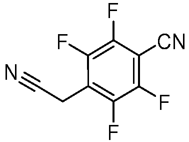 | 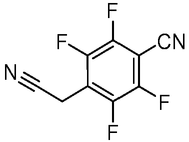 | -5.19 |
| A60 | 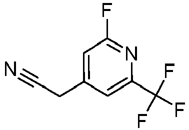 | 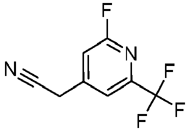 | 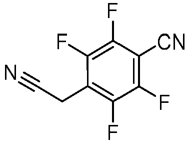 | -5.19 |
| A61 | 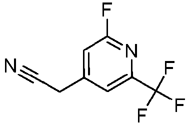 | 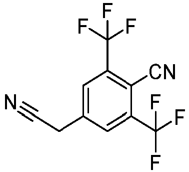 | 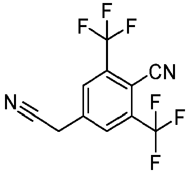 | -5.30 |
| A62 | 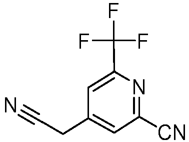 | 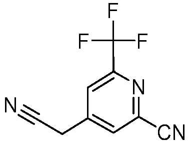 | 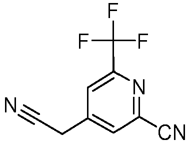 | -5.58 |
| A63 | 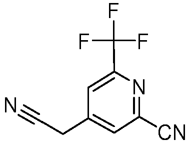 | 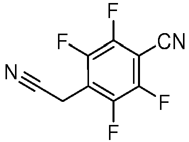 | 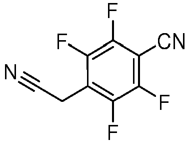 | -5.32 |
| A64 | 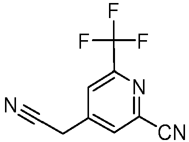 | 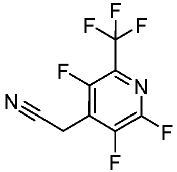 | 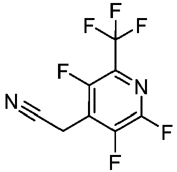 | -5.39 |
| A65 | 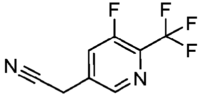 | 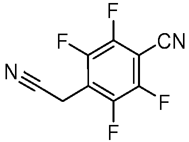 | 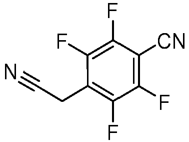 | -5.14 |
| A66 | 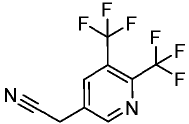 | 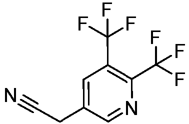 | 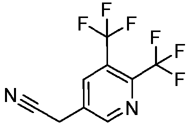 | -5.13 |
| A67 | 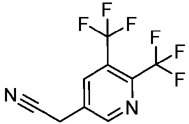 | 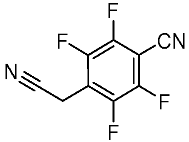 | 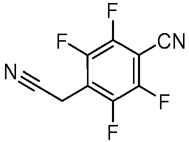 | -5.17 |
| A68 | 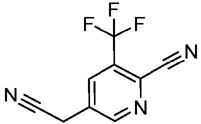 | 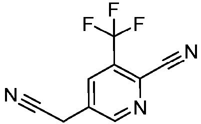 | 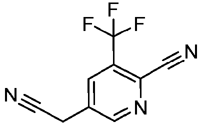 | -5.36 |
| A69 | 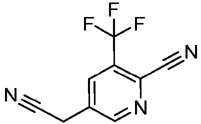 | 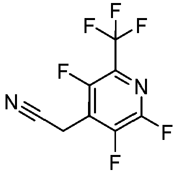 | 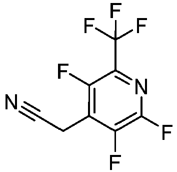 | -5.31 |
| A70 | 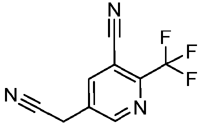 | 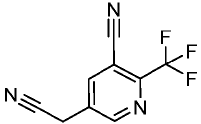 | 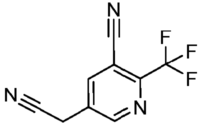 | -5.37 |
| A71 | 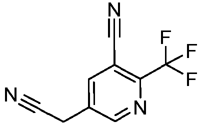 | 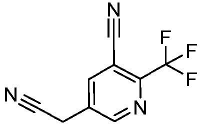 | 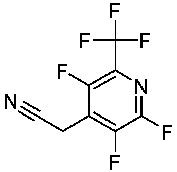 | -5.34 |
| A72 | 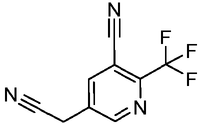 | 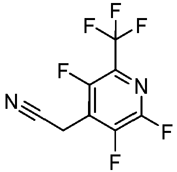 | 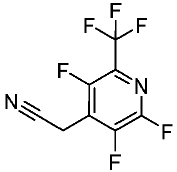 | -5.32 |
| A73 | 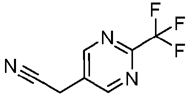 | 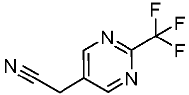 | 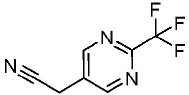 | -5.15 |
| A74 | 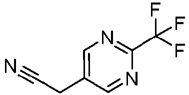 | 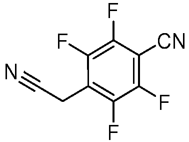 | 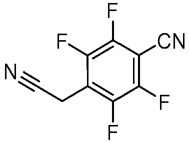 | -5.18 |
| A75 | 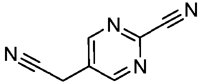 | 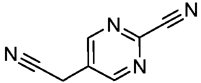 | 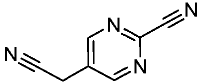 | -5.42 |
| A76 | 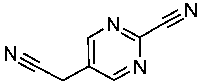 | 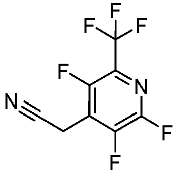 | 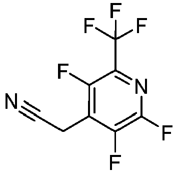 | -5.33 |
| A77 | 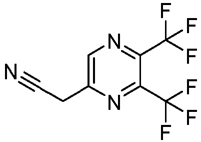 | 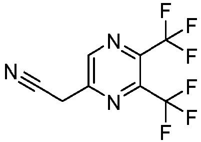 | 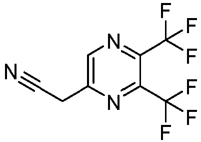 | -5.28 |
| A78 | 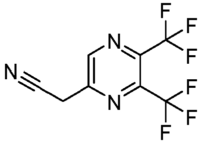 | 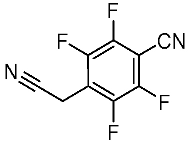 | 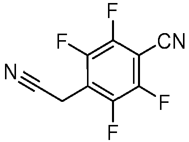 | -5.22 |
| A79 | 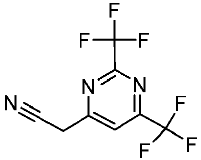 | 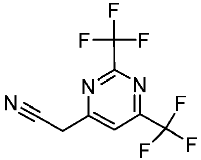 | 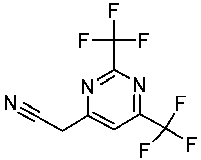 | -5.50 |
| A80 | 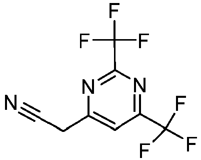 | 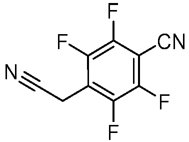 | 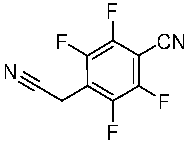 | -5.29 |
| A81 | 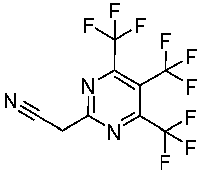 | 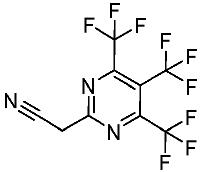 | 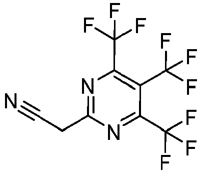 | -5.40 |
| A82 | 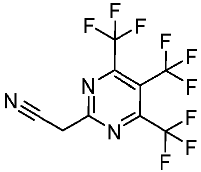 | 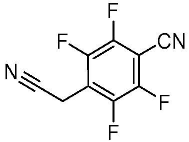 | 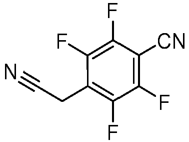 | -5.26 |

Additionally two comparative compounds 1 and 2 were used, the structure of which is shown in Table 3.

**Table 3: Structure of the comparative examples 1 and 2**

| | A¹ | A² | A³ |
|---|---|---|---|
| Comparative compound 1 (CC1) | 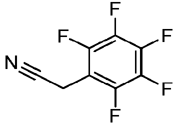 | 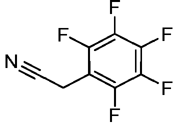 | 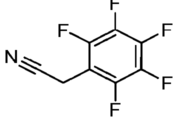 |
| Comparative compound 2 (CC2) | 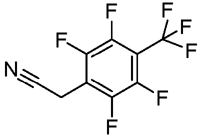 | 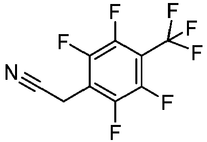 | 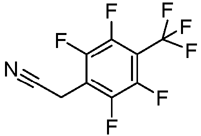 |

In Table 4 are shown some physical properties of several inventive compounds and the two comparative compounds 1 and 2:

**Table 4: Physical properties of three compounds of formula (I) and comparative compounds 1 and 2**

| | LUMO [eV] | TGA5% [°C] | Tg [°C] |
|---|---|---|---|
| Comparative compound 1 (CC1) | -4.58 | 264 | 65 |
| Comparative compound 2 (CC2) | -4.90 | 270 | 78 |
| Compound A1 | -5.36 | 419 | n/obs |
| Compound A2 | -5.30 | 381 | 130 |
| Compound A30 | -5.24 | 403 | n/obs |
| Compound A55 | -5.24 | 396 | n/obs |
| Compound A76 | -5.19 | 339 | 107 |

As can be seen in Table 4, compounds of formula (I) may have improved LUMO values, reduced volatility as determined by TGA5% and/or improved glass transition temperatures.

In Table 5 are shown data for bottom emission devices. The light is emitted through the transparent anode and substrate.

In comparative example 1-1 the p-CGL comprises a substantially covalent matrix compound of formula F11 and comparative compound CC1. The LUMO of CC1 is -4.58 eV and the Tg is 65 °C, see Table 4. The operating voltage U is 9.83 V. Lifetime and voltage rise over time were not determined due to the high operating voltage.

In comparative example 1-2, the p-CGL comprises a substantially covalent matrix compound of formula F11 and comparative compound CC2. The LUMO of CC2 is -4.9 eV the Tg is 78 °C, see Table 4. The operating voltage U is improved to 8.04 V. The lifetime is 144 hours and the voltage rise over time (100-1h) is 0.09 V and the voltage rise over time (400-1h) is 0.193 V.

In example 1-1, the p-CGL comprises a substantially covalent matrix compound of formula F11 and compound of formula (I) A1. The LUMO of A1 is -5.36 eV, see Table 4. The operating voltage U is improved to 7.58 V. The lifetime is improved to 153 hours and the voltage rise over time (100-1h) is improved to 0.059 V and the voltage rise over time (400-1h) is improved to 0.13 V.

In example 1-2, the amount of compound of formula (I) A1 in the p-CGL has been increased from 5 to 10 vol.-% compared to example 1-1. The operating voltage U is further improved to 7.38 V. The lifetime is unchanged at 153 hours and the voltage rise over time (100-1h) is 0.054 V and the voltage rise over time (400-1h) is 0.124 V.

In example 1-3, the p-CGL comprises a substantially covalent matrix compound of formula F11 and compound of formula (I) A55. The LUMO of A55 is -5.24 eV, see Table 4. The operating voltage U is improved to 7.47 V. The lifetime is improved to 156 hours and the voltage rise over time (100-1h) is improved to 0.062 V and the voltage rise over time (400-1h) is improved to 0.135 V.

In example 1-4, the p-CGL comprises a substantially covalent matrix compound of formula F11 and compound of formula (I) A30. The LUMO of A30 is -5.24 eV, see Table 4. The operating voltage U is improved to 7.45 V. The lifetime is improved to 151 hours and the voltage rise over time (100-1h) is improved to 0.086 V and the voltage rise over time (400-1h) is improved to 0.185 V.

In example 1-5, the p-CGL comprises a substantially covalent matrix compound of formula F11 and compound of formula (I) A76. The LUMO of A76 is -5.19 eV, see Table 4. The operating voltage U is improved to 7.43 V. The lifetime is improved to 157 hours and the voltage rise over time (100-1h) is improved to 0.049 V and the voltage rise over time (400-1h) is improved to 0.122 V.

In comparative example 1-3 the p-CGL and the HIL comprise a substantially covalent matrix compound of formula F11 and comparative compound CC1. The operating voltage U is > 12 V. Lifetime and voltage rise over time were not determined due to the high operating voltage.

In comparative example 1-4, the p-CGL and the HIL comprise a substantially covalent matrix compound of formula F11 and comparative compound CC2. The operating voltage U is improved to 8.11 V. The lifetime is 150 hours and the voltage rise over time (100-1h) is 0.092 V and the voltage rise over time (400-1h) is 0.175 V.

In Example 1-6, the p-CGL and the HIL comprise a substantially covalent matrix compound of formula F11 and compound of formula (I) A1. The operating voltage U is improved to 7.61 V. The lifetime is improved to 159 hours and the voltage rise over time (100-1h) is 0.077 V and the voltage rise over time (400-1h) is 0.166 V.

In Example 1-7, the amount of compound of formula (I) A1 in the p-CGL has been increased from 5 to 10 vol.-% compared to example 1-3. The operating voltage U is further improved to 7.36 V. The lifetime is further improved to 168 hours and the voltage rise over time (100-1h) is improved to 0.054 V and the voltage rise over time (400-1h) is improved to 0.134 V.

In Example 1-8, the p-CGL and the HIL comprise a substantially covalent matrix compound of formula F11 and compound of formula (I) A2. The operating voltage U is improved to 7.61 V. The lifetime is improved to 157 hours and the voltage rise over time (100-1h) is 0.083 V and the voltage rise over time (400-1h) is 0.204 V.

In Example 1-9, the amount of compound of formula (I) A2 in the p-CGL has been increased from 5 to 10 vol.-% compared to Example 1-8. The operating voltage U is further improved to 7.39 V. The lifetime is further improved to 159 hours and the voltage rise over time (100-1h) is improved to 0.063 V and the voltage rise over time (400-1h) is improved to 0.173 V.

In Table 6 are shown data for top emission devices. The light is emitted through the transparent cathode.

In comparative example 2-1 the p-CGL comprises a substantially covalent matrix compound of formula F11 and comparative compound CC1. The operating voltage U is > 12 V.

In comparative example 2-2, the p-CGL comprises a substantially covalent matrix compound of formula F11 and comparative compound CC2. The operating voltage U is improved to 6.77 V.

In example 2-1, the p-CGL comprises a substantially covalent matrix compound of formula F11 and compound of formula (I) A1. The operating voltage U is further improved to 6.26 V.

In example 2-2, the amount of compound of formula (I) A1 in the p-CGL has been increased from 5 to 10 vol.-% compared to example 1-1. The operating voltage U is further improved to 6.13 V.

In example 2-3, the p-CGL comprises a substantially covalent matrix compound of formula F11 and compound of formula (I) A55. The operating voltage U is improved to 6.24 V compared to comparative example 2-2.

In example 2-4, the p-CGL comprises a substantially covalent matrix compound of formula F11 and compound of formula (I) A30. The operating voltage U is improved to 6.17 V compared to comparative example 2-2.

In example 2-5, the p-CGL comprises a substantially covalent matrix compound of formula F11 and compound of formula (I) A76. The operating voltage U is improved to 6.08 V compared to comparative example 2-2.

In comparative Example 2-3 the p-CGL and the HIL comprise a substantially covalent matrix compound of formula F11 and comparative compound CC1. The operating voltage U is > 12 V.

In comparative Example 2-4, the p-CGL and the HIL comprise a substantially covalent matrix compound of formula F11 and comparative compound CC2. The operating voltage U is improved to 6.92 V.

In Example 2-6, the p-CGL and the HIL comprise a substantially covalent matrix compound of formula F11 and compound of formula (I) A1. The operating voltage U is further improved to 6.36 V.

In Example 2-7, the amount of compound of formula (I) A1 in the p-CGL has been increased from 5 to 10 vol.-% compared to example 1-3. The operating voltage U is further improved to 6.22 V.

A low operating voltage U may be beneficial for reduced power consumption and improved battery life, in particular in mobile devices.

The particular combinations of elements and features in the above detailed embodiments are exemplary only.

As those skilled in the art will recognize, variations, modifications, and other implementations of what is described herein can occur to those of ordinary skill in the art without departing from the scope of the invention as claimed. Accordingly, the foregoing description is by way of example only and is not intended as limiting. In the claims, the word "comprising" does not exclude other elements or steps, and the indefinite article "a" or "an" does not exclude a plurality. The mere fact that certain measures are recited in mutually different dependent claims does not indicate that a combination of these measured cannot be used to advantage. The invention's scope is defined in the following claims. Furthermore, reference signs used in the description and claims do not limit the scope of the invention as claimed.

**Table 5: Organic electronic devices comprising a transparent anode, a p-type charge generation layer (p-CGL) comprising a compound of formula (I) and a substantially organic matrix compound**

| | HIL | | | p-CGL | | | | | | |
|---|---|---|---|---|---|---|---|---|---|---|
| | Matrix compund | Compound of formula (I) | Amount compound of formula (I) [ vol.-%] | Matrix compund | Compound of formula (I) | Amount compound of formula (I) [ vol.-%] | U at 15 mA/cm² [V] | LT at 30 mA/cm² [h] | U rise (100-1h) at 30mA/cm² [V] | U rise (400-1h) at 30mA/cm² [V] |
| Comparative example 1-1 | F11 | CC3 | 5 | F11 | CC1 | 5 | 9.83 | - | - | - |
| | F11 | | | | | | | | | |
| Comparative example 1-2 | F11 | CC3 | 5 | F11 | CC2 | 5 | 8.04 | 144 | 0.09 | 0.193 |
| Example 1-1 | F11 | CC3 | 5 | F11 | A1 | 5 | 7.58 | 153 | 0.059 | 0.13 |
| Example 1-2 | F11 | CC3 | 5 | F11 | A1 | 10 | 7.38 | 153 | 0.054 | 0.124 |
| Example 1-3 | F11 | CC3 | 5 | F11 | A55 | 10 | 7.47 | 156 | 0.062 | 0.135 |
| Example 1-4 | F11 | CC3 | 5 | F11 | A30 | 10 | 7.45 | 151 | 0.086 | 0.185 |
| Example 1-6 | F11 | CC3 | 5 | F11 | A76 | 10 | 7.43 | 157 | 0.049 | 0.122 |
| Comparative example 1-3 | F11 | CC1 | 5 | F11 | CC1 | 5 | >12 | - | - | - |
| Comparative example 1-4 | F11 | CC2 | 5 | F11 | CC2 | 5 | 8.11 | 150 | 0.092 | 0.175 |
| Example 1-6 | F11 | A1 | 5 | F11 | A1 | 5 | 7.61 | 159 | 0.077 | 0.166 |
| Example 1-7 | F11 | A1 | 5 | F11 | A1 | 10 | 7.36 | 168 | 0.054 | 0.134 |
| Example 1-8 | F11 | A2 | 5 | F11 | A2 | 5 | 7.61 | 157 | 0.083 | 0.204 |
| Example 1-9 | F11 | A2 | 5 | F11 | A2 | 10 | 7.39 | 159 | 0.063 | 0.173 |

**Table 6: Organic electronic devices comprising a transparent cathode, a p-type charge generation layer (p-CGL) comprising a compound of formula (I) and a substantially organic matrix compound**

| | HIL | | | p-CGL | | | |
|---|---|---|---|---|---|---|---|
| | Matrix compund | Compound of formula (I) | Amount compound of formula (I) [ vol.-%] | Matrix compund | Compound of formula (I) | Amount compound of formula (I) [ vol.-%] | U at 15 mA/cm² [V] |
| Comparative example 2-1 | F11 | CC3 | 5 | F11 | CC1 | 5 | >12 |
| Comparative example 2-2 | F11 | CC3 | 5 | F11 | CC2 | 5 | 6.77 |
| Example 2-1 | F11 | CC3 | 5 | F11 | A1 | 5 | 6.26 |
| Example 2-2 | F11 | CC3 | 5 | F11 | A1 | 10 | 6.13 |
| Example 2-3 | F11 | CC3 | 5 | F11 | A55 | 10 | 6.24 |
| Example 2-4 | F11 | CC3 | 5 | F11 | A30 | 10 | 6.17 |
| Example 2-5 | F11 | CC3 | 5 | F11 | A76 | 10 | 6.08 |
| Comparative Example 2-3 | F11 | CC1 | 5 | F11 | CC1 | 5 | >12 |
| Comparative Example 2-4 | F11 | CC2 | 5 | F11 | CC2 | 5 | 6.92 |
| Example 2-6 | F11 | A1 | 5 | F11 | A1 | 5 | 6.36 |
| Example 2-7 | F11 | A1 | 5 | F11 | A1 | 10 | 6.22 |

## Claims

1. An organic electronic device comprising an anode layer (120), a cathode layer (190) and a charge generation layer, wherein the charge generation layer comprises a p-type charge generation layer (135) and a n-type charge generation player (185), wherein the p-type charge generation layer comprises a compound of formula (I) 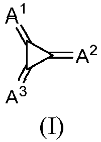
whereby A¹ is selected from formula (II) 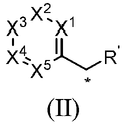
X¹ is selected from CR¹ or N;
X² is selected from CR² or N;
X³ is selected from CR³ or N;
X⁴ is selected from CR⁴ or N;
X⁵ is selected from CR⁵ or N;
whereby when any of R¹, R², R³, R⁴ and R⁵ is present, then the corresponding X¹, X², X³, X⁴ and X⁵ is not N;
wherein "*" denotes the binding position;
with the proviso that
- R¹ (if present) is selected from D or H; and
- at least one of R², R³, R⁴ is present and for each of the R², R³, R⁴ that are present, the corresponding σ^{x} is > 0. 33, with σ^{x} being the Hammett constant of R^{x};
A² and A³ are independently selected from formula (III) 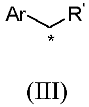
wherein Ar is independently selected from substituted or unsubstituted C₆ to C₁₈ aryl and substituted or unsubstituted C₂ to C₁₈ heteroaryl, wherein the substituents on Ar are independently selected from CN, partially or perfluorinated C₁ to C₆ alkyl, halogen, Cl, F, D;
and R' is selected from Ar, substituted or unsubstituted C₆ to C₁₈ aryl or C₃ to C₁₈ heteroaryl, partially flurorinated or perfluorinated C₁ to C₈ alkyl, halogen, F or CN; whereby R⁵ (if present) is selected from D or H.

2. The device of claim 1, whereby the p-type charge generation layer comprises a compound of formula (IV) 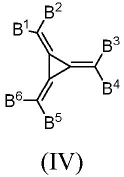 whereby B¹ is selected from formula (V) 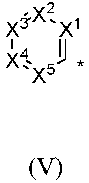 B³ and B⁵ are Ar and B², B⁴ and B⁶ are R'.

3. The device of claim 1 or 2, whereby σ^{total} > 0.5, with σ^{total} = σ² + σ³ + σ⁴ and σ^{x} being the Hammett constant of R^{x} (if present).

4. The device of any of the claims 1 to 3, whereby the compound of formula (I) comprises less than nine cyano moieties.

5. The device of any of the claims 1 to 4, whereby the LUMO of the compound of formula (I) is ≤ -5.05 eV, when calculated with the program package TURBOMOLE V6.5 by applying the hybrid functional B3LYP with a 6-31G* basis set in the gas phase.

6. The device of any of the claims 1 to 5, whereby σ^{total} is > 0.6.

7. The organic electronic device of any of the claims 1 to 6, whereby the p-type charge generation layer comprises a composition comprising a compound of formula (IV) and at least one compound of formula (IVa) to (IVd)

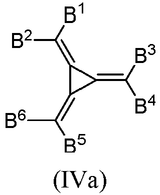

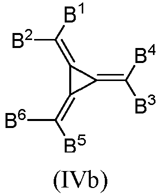

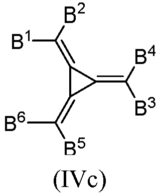

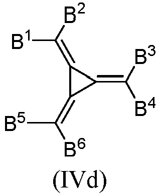

8. The organic electronic device of any of the claims 1 to 7, wherein the p-type charge generation layer further comprises a substantially covalent matrix compound.

9. The organic electronic device of any of the claims 1 to 8, further comprising a hole injection layer, wherein the hole injection layer is arranged between the anode layer and the charge generation layer and whereby the hole injection layer comprises a compound of formula (I) or (IV).

10. The organic electronic device of any of the claims 1 to 9, whereby the p-type charge generation layer and the hole injection layer comprise an identical compound of formula (I) or (IV).

11. The organic electronic device of any of the claims 1 to 10, whereby the p-type charge generation layer and the hole injection layer comprise an identical substantially covalent matrix compound.

12. The organic electronic device of any of the claims 1 to 11, whereby the electronic organic device is an electroluminescent device, preferably an organic light emitting diode.

13. A display device comprising an organic electronic device according to any of the claims 1 to 12.

14. A compound of formula (I) of claim 1 having less than nine cyano moieties and a LUMO of ≤ -5.05 eV, when calculated with the program package TURBOMOLE V6.5 by applying the hybrid functional B3LYP with a 6-31G* basis set in the gas phase.

## Patentansprüche

1. Organische elektronische Vorrichtung, umfassend eine Anodenschicht (120), eine Kathodenschicht (190) und eine Ladungserzeugungsschicht, wobei die Ladungserzeugungsschicht eine Ladungserzeugungsschicht vom p-Typ (135) und eine Ladungserzeugungsschicht vom n-Typ (185) umfasst, wobei die Ladungserzeugungsschicht vom p-Typ eine Verbindung der Formel (I) 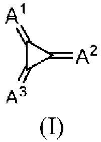
umfasst, wobei A¹ ausgewählt ist aus Formel (II) 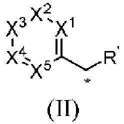
X¹ aus CR¹ oder N ausgewählt ist;
X² aus CR² oder N ausgewählt ist;
X³ aus CR³ oder N ausgewählt ist;
X⁴ aus CR⁴ oder N ausgewählt ist;
X⁵ aus CR⁵ oder N ausgewählt ist;
wobei dann, wenn eines von R¹, R², R³, R⁴ und R⁵ vorliegt, das entsprechende X¹, X², X³, X⁴ und X⁵ nicht für N steht; wobei "*" die Bindungsposition bezeichnet;
mit der Maßgabe, dass
- R¹ (falls vorhanden) aus D oder H ausgewählt ist und
- mindestens eines von R², R³ und R⁴ vorliegt und für jedes der vorliegenden R², R³ und R⁴ das entsprechende σ^{x} > 0,33 ist, wobei σ^{x} die Hammett-Konstante von R^{x} ist;
A² und A³ unabhängig aus Formel (III) 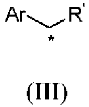
ausgewählt sind, wobei Ar unabhängig aus substituiertem oder unsubstituiertem C₆- bis C₁₈-Aryl und substituiertem oder unsubstituiertem C₂- bis C₁₈-Heteroaryl ausgewählt ist, wobei die Substituenten an Ar unabhängig aus CN, teil- oder perfluoriertem C₁- bis C₆-Alkyl, Halogen, Cl, F, D ausgewählt sind;
und R' aus Ar, substituiertem oder unsubstituiertem C₆-bis C₁₈-Aryl oder C₃-bis C₁₈-Heteroaryl, teil- oder perfluoriertem C₁- bis C₈-Alkyl, Halogen, F oder CN ausgewählt ist;
wobei R⁵ (falls vorhanden) aus D oder H ausgewählt ist.

2. Vorrichtung nach Anspruch 1, wobei die Ladungserzeugungsschicht vom p-Typ eine Verbindung der Formel (IV) 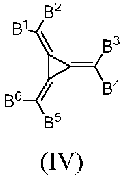 umfasst, wobei B¹ aus Formel (V) 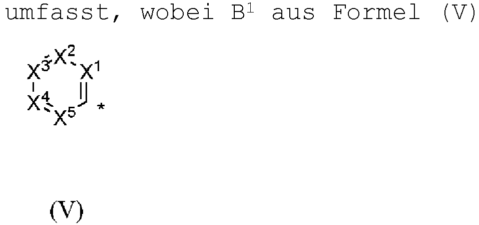 ausgewählt ist, B³ und B⁵ für Ar stehen und B², B⁴ und B⁶ für R' stehen.

3. Vorrichtung nach Anspruch 1 oder 2, wobei σ^{gesamt} > 0,5 ist, wobei σ^{gesamt} = σ² + σ³ + σ⁴ und σ^{x} die Hammett-Konstante von R^{x} (falls vorhanden) ist.

4. Vorrichtung nach einem der Ansprüche 1 bis 3, wobei die Verbindung der Formel (I) weniger als neun Cyanogruppierungen umfasst.

5. Vorrichtung nach einem der Ansprüche 1 bis 4, wobei das LUMO der Verbindung der Formel (I) bei Berechnung mit dem Programmpaket TURBOMOLE V6.5 durch Anwenden des Hybridfunktionals B3LYP mit einem 6-31G*-Basissatz in der Gasphase ≤ -5,05 eV ist.

6. Vorrichtung nach einem der Ansprüche 1 bis 5, wobei σ^{gesamt} > 0,6 ist.

7. Organische elektronische Vorrichtung nach einem der Ansprüche 1 bis 6, wobei die Ladungserzeugungsschicht vom p-Typ eine Zusammensetzung umfasst, die eine Verbindung der Formel (IV) und mindestens eine Verbindung der Formel (IVa) bis (IVd) umfasst

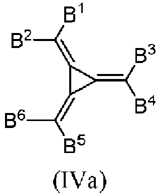

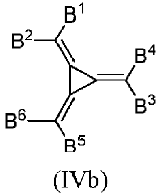

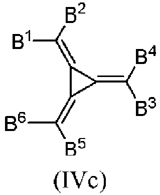

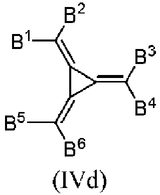

8. Organische elektronische Vorrichtung nach einem der Ansprüche 1 bis 7, wobei die Ladungserzeugungsschicht vom p-Typ ferner eine weitgehend kovalente Matrixverbindung umfasst.

9. Organische elektronische Vorrichtung nach einem der Ansprüche 1 bis 8, ferner umfassend eine Lochinjektionsschicht, wobei die Lochinjektionsschicht zwischen der Anodenschicht und der Ladungserzeugungsschicht angeordnet ist und wobei die Lochinjektionsschicht eine Verbindung der Formel (I) oder (IV) umfasst.

10. Organische elektronische Vorrichtung nach einem der Ansprüche 1 bis 9, wobei die Ladungserzeugungsschicht vom p-Typ und die Lochinjektionsschicht eine identische Verbindung der Formel (I) oder (IV) umfassen.

11. Organische elektronische Vorrichtung nach einem der Ansprüche 1 bis 10, wobei die Ladungserzeugungsschicht vom p-Typ und die Lochinjektionsschicht eine identische weitgehend kovalente Matrixverbindung umfassen.

12. Organische elektronische Vorrichtung nach einem der Ansprüche 1 bis 11, wobei es sich bei der elektronischen organischen Vorrichtung um eine Elektrolumineszenzvorrichtung, vorzugsweise eine organische Leuchtdiode, handelt.

13. Anzeigevorrichtung, umfassend eine organische elektronische Vorrichtung gemäß einem der Ansprüche 1 bis 12.

14. Verbindung der Formel (I) nach Anspruch 1 mit weniger als neun Cyanogruppierungen und einem LUMO bei Berechnung mit dem Programmpaket TURBOMOLE V6.5 durch Anwenden des Hybridfunktionals B3LYP mit einem 6-31G*-Basissatz in der Gasphase von ≤ -5,05 eV.

## Revendications

1. Dispositif électronique organique comprenant une couche anodique (120), une couche cathodique (190) et une couche de génération de charges, dans lequel la couche de génération de charges comprend une couche de génération de charges de type p (135) et une couche de génération de charges de type n (185), la couche de génération de charges de type p comprenant un composé de formule (I) 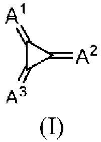
A¹ étant choisi parmi les groupes de formule (II) 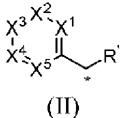
X¹ étant choisi entre CR¹ et N ;
X² étant choisi entre CR² et N ;
X³ étant choisi entre CR³ et N ;
X⁴ étant choisi entre CR⁴ et N ;
X⁵ étant choisi entre CR⁵ et N ;
lorsque l'un quelconque de R¹, R², R³, R⁴ et R⁵ est présent, alors les X¹, X², X³, X⁴ et X⁵ correspondants n'étant pas N ;
« * » désignant la position de liaison ;
à condition que
- R¹ (s'il est présent) soit choisi entre D et H ; et
- au moins l'un de R², R³, R⁴ soit présent et que pour chacun des R², R³, R⁴ qui sont présents, la σ^{x} correspondante soit > 0,33, σ^{x} étant la constante de Hammett de R^{x} ;
A² et A³ étant indépendamment choisis parmi les groupes de formule (III) 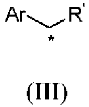
Ar étant indépendamment choisi entre aryle en C₆ à C₁₈ substitué ou non substitué et hétéroaryle en C₂ à C₁₈ substitué ou non substitué, les substituants sur Ar étant indépendamment choisis parmi CN, alkyle en C₁ à C₆ partiellement fluoré ou perfluoré, halogène, Cl, F, D ; et R' étant choisi parmi Ar, aryle en C₆ à C₁₈ ou hétéroaryle en C₃ à C₁₈ substitué ou non substitué, alkyle en C₁ à C₈ partiellement fluoré ou perfluoré, halogène, F ou CN ;
R⁵ (s'il est présent) étant choisi entre D et H.

2. Dispositif selon la revendication 1, dans lequel la couche de génération de charges de type p comprend un composé de formule (IV) 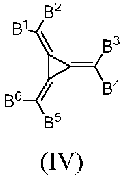
B¹ étant choisi parmi les groupes de formule (V) 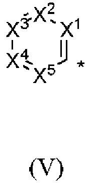
B³ et B⁵ étant Ar et B², B⁴ et B⁶ étant R' .

3. Dispositif selon la revendication 1 ou 2, dans lequel σ^{totale} est > 0,5, avec σ^{totale} = σ² + σ³ + σ⁴ et σ^{x} étant la constante de Hammett de R^{x} (s'il est présent).

4. Dispositif selon l'une quelconque des revendications 1 à 3, dans lequel le composé de formule (I) comprend moins de neuf fractions cyano.

5. Dispositif selon l'une quelconque des revendications 1 à 4, la LUMO du composé de formule (I) étant ≤ -5,05 eV, lorsqu'elle est calculée avec le progiciel TURBOMOLE V6.5 par application de la fonctionnelle hybride B3LYP avec une base 6-31G* établie en phase gazeuse.

6. Dispositif selon l'une quelconque des revendications 1 à 5, dans lequel σ^{totale} est > 0,6.

7. Dispositif électronique organique selon l'une quelconque des revendications 1 à 6, dans lequel la couche de génération de charges de type p comprend une composition comprenant un composé de formule (IV) et au moins un composé de formule (IVa) à (IVd)

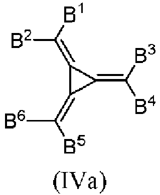

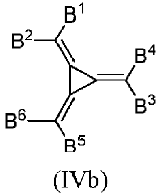

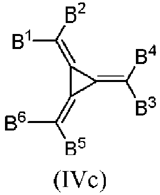

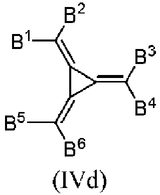

8. Dispositif électronique organique selon l'une quelconque des revendications 1 à 7, dans lequel la couche de génération de charges de type p comprend en outre un composé matrice essentiellement covalent.

9. Dispositif électronique organique selon l'une quelconque des revendications 1 à 8, comprenant en outre une couche d'injection de trous, dans lequel la couche d'injection de trous est disposée entre la couche anodique et la couche de génération de charges et dans lequel la couche d'injection de trous comprend un composé de formule (I) ou (IV).

10. Dispositif électronique organique selon l'une quelconque des revendications 1 à 9, dans lequel la couche de génération de charges de type p et la couche d'injection de trous comprennent un composé de formule (I) ou (IV) identique.

11. Dispositif électronique organique selon l'une quelconque des revendications 1 à 10, dans lequel la couche de génération de charges de type p et la couche d'injection de trous comprennent un composé matrice essentiellement covalent identique.

12. Dispositif électronique organique selon l'une quelconque des revendications 1 à 11, le dispositif électronique organique étant un dispositif électroluminescent, de préférence une diode électroluminescente organique.

13. Dispositif d'affichage comprenant un dispositif électronique organique selon l'une quelconque des revendications 1 à 12.

14. Composé de formule (I) selon la revendication 1 ayant moins de neuf fractions cyano et une LUMO ≤ - 5,05 eV, lorsqu'elle est calculée avec le progiciel TURBOMOLE V6.5 par application de la fonctionnelle hybride B3LYP avec une base 6-31G* établie en phase gazeuse.
